# EUROPEAN PATENT APPLICATION

(11) **EP 4 616 905 A1**
(43) Date of publication of application: **17.09.2025**
(21) Application number: 24162887.4
(22) Date of filing: 12.03.2024
(51) Int. Cl.: A61P 11/00, C12N 15/11

(54) **USE OF INHIBITORS OF MICRORNAS IN THE TREATMENT OF LUNG DISEASES**

(71) Applicant: International Centre For Genetic Engineering And Biotechnology - ICGEB, 34149 Trieste (IT)
(72) Inventor: BRAGA, Luca, 34149 Trieste (TS) (IT); VOLPE, Maria Concetta, 34149 Trieste (TS) (IT); ZANDOMENEGO, Giulia, 34149 Trieste (TS) (IT)
(74) Representative: Pace Napoleone, Maria

(57) **Abstract**

The present invention refers to an agent for use in the prevention and/or treatment of lung pathological conditions associated with loss of alveolar epithelial type II to type I transdifferentiation capacity and/or with loss of lung regenerative capacity and/or for use in promoting lung regeneration in a subject with a lung pathological condition, said agent being selected from the group consisting of: an inhibitor of miR-210-3p and an inhibitor of miR-155-5p or a combination thereof.

## Description

### FIELD OF THE INVENTION

The present invention relates to at least one agent selected from inhibitors of miR-210-3p or miR-155-5p or combinations thereof for use in the treatment and/or prevention of lung pathological conditions associated with senescence of alveolar epithelial type II and/or type I cells and/or loss of alveolar epithelial type II to type I trans-differentiation capacity and/or with loss of lung regenerative capacity and/or for use in promoting lung regeneration in a subject with a lung condition, wherein the pathological condition is preferably selected from the group of interstitial lung diseases, chronic obstructive pulmonary diseases, post-acute respiratory distress syndrome, post-acute COVID-19 syndrome, said interstitial lung disease being preferably selected from the group consisting of idiopathic pulmonary fibrosis, non-specific interstitial pneumonia, desquamative interstitial pneumonia, respiratory bronchiolitis-ILD, cryptogenic organizing pneumonia and acute interstitial pneumonia.

### BACKGROUND OF THE INVENTION

Idiopathic Pulmonary Fibrosis (IPF) is a chronic Interstitial Lung Disease (ILDs). ILDs with either known or unknown (Idiopathic) cause are characterized by gradual fibrotic scarring of the lungs that leads to the anatomopathological and radiological pattern of usual interstitial pneumonia (UIP) and, eventually, to death. Idiopathic Pulmonary Fibrosis (IPF) represents the most common encountered ILD in clinical practice, ranging from 0.9 to 14 cases/100.000/y in incidence worldwide ¹. IPF is considered an aging-associated disease, with increase in both incidence and prevalence in aging population. Indeed, the IPF occurs mainly at 50-70 years of age ¹. At the cellular level, pulmonary fibrosis can be considered as a compensatory fibrotic response to impaired lung regeneration following chronic or acute alveolar injury. Several studies suggest a central role of alveolar type II epithelial cells (ATII) cells in lung regeneration ²⁻⁴. Physiologically, the turnover of alveolar type I epithelial cells (ATI) cells is supported by the trans-differentiation of ATII cells into ATI cells; ATII cells are also responsible for surfactant production ². It has been shown that both extensive ATII loss or the accumulation of ATII to ATI aberrant intermediates, after lung injury, may trigger inadequate repair and onset of IPF ^{2,5-12}.

The incapability of ATIIs to transdifferentiate into ATIs is recapitulated also in the mouse model of bleomycin-induced lung fibrosis ⁹, which currently represents the most accurate mouse preclinical model of IPF ¹³.

Bleomycin is a chemotherapeutic agent, which acts by causing single and double-strand DNA breaks in cellular DNA, thus leading to cell permanent cell cycle arrest and accumulation in the lungs of p21, p16 and beta-gal positive cells. In addition, ageing processes have been shown to induce a dysfunctional phenotype in ATII cells, characterized by aberrant secretion of profibrotic and pro-inflammatory senescence-associated factors SASP ¹⁴⁻¹⁶.

Similarly, multiple genes related with telomere maintenance such as TERT, TERC, TINF2, DKC1, RTEL1, PARN and NAF1 have been found to be mutated in the 25% of patients with familial IPF, thus further confirming the pathological role of cellular senescence in disease progression ¹⁷.

The bleomycin mouse presents some differences with human IPF, with the most significant being the progressive and spontaneous resolution of fibrosis in young mice, at 60 days after bleomycin administration ¹⁸.

Recent literature demonstrated that studies of bleomycin induced fibrosis in aged male mice (18 weeks) result to be the most clinically relevant for preclinical study for human ¹³. Unlike young mice, aged mice haven't been shown to undergo spontaneous resolution of bleomycin induces pulmonary fibrosis ¹⁹, thus better reflecting human IPF. In support of connection between IPF and cellular senescence, it has been shown that selective ablation of senescent cells using senolytics alleviates IPF-related dysfunction in bleomycin mouse model, as well as in IPF patients ^{14,20,21}.

MicroRNAs (miRNAs or miRs) are evolutionarily conserved small noncoding RNAs that regulate gene expression at the post-transcriptional level. In animal cells, repression of gene expression by microRNAs is achieved by base-pairing to partially complementary sequences present mostly in 3' UTRs of target messenger RNAs (mRNAs), resulting in translation repression, mRNA degradation, or both ²². The microRNA seed sequence, essential for the binding of the microRNA to the mRNA, is a conserved sequence, which is generally situated at positions 2-8 from the microRNA 5'-end ²³. Even though base pairing of microRNA and its target mRNA is not perfect, the region corresponding to the seed sequence usually has to be perfectly complementary. Thus, the seed sequence is the primary specificity determinant for target selection. The small size of the seed sequence means that a single microRNA can regulate many, even hundreds, different genes. MicroRNAs are genome-encoded sequences generally transcribed by RNA polymerase II into primary microRNAs (pri-microRNAs). The pri-microRNAs are then sequentially processed by two endonucleases of the RNAse III family: in the nucleus, Drosha processes the pri-microRNA into a precursor microRNA (pre-microRNA) of approximately 60-80 nucleotides, after which the pre-microRNA is further processed, in the cytoplasm, by Dicer to form a duplex containing two strands of about 19-23 nucleotides. The microRNA duplex is then unwound, and the mature microRNA is incorporated into the RNAinduced silencing complex (RISC), containing, among others, Argonaute and GW182 proteins essential for the silencing by microRNAs ²⁴.

MicroRNAs perform relevant roles in maintaining lung functions and homeostasis ²⁵. They are involved in several cellular processes which occur in the lung, such as cell proliferation, cell differentiation, apoptosis, and inflammatory responses. The importance of miRNAs in lung development has been demonstrated by Harris et al., who showed that conditional knock out of Dicer1 in the murine lung epithelium, in both neonatal and adult lung, leads to an improper branching morphogenesis and a severe ECM production with fibrosis development ²⁶. Several miRNAs have been discovered to regulate specific processes in lung repair, such as miR-26, miR-142, miR-17, miR-92 that play crucial role in ATI and ATII cell homeostasis ²⁷. On the contrary, there are several miRNAs that have been involved in the pathogenesis of lung diseases, such as inflammation and viral infections, lung carcinogenesis, pulmonary allergy, asthma, chronic obstructive pulmonary disease and IPF ²⁸.

Other microRNAs such as miR-21, miR-29, miR-153 are abnormally regulated in IPF lungs ¹⁹. In normal lung, miR-21 is downregulated, leading to upregulation of Smad-7. Smad-7 inhibits the proliferation of interstitial fibroblasts. But in IPF lung, there is an increase in miR-21 expression, thus an altered control of smad-7 ²⁹. MicroRNA-29 is decreased in the IPF lung and the restoration of miR-29 physiological levels showed protective effect in mouse model of pulmonary fibrosis ²⁸. MicroRNA-29 has been shown to negatively regulate the production of collagen, thus its downregulation causes excessive ECM deposition, contributing to the development of fibrosis ³⁰. Another miRNA involved in the development of IPF is miR-153. Normally, this miRNA targets TGF-β1, regulating the proliferation and activation of fibroblasts and the ECM deposition. However, its expression levels are reduced in fibrotic lungs ³¹.

MicroRNA-based therapeutic approaches can be subdivided into two fundamental groups: microRNA replacement and miRNA inhibition ³². The first system is used to rescue the microRNA effect, decreasing the expression of specific microRNA target. The second approach aims at reducing endogenous miRNA levels and herefore increasing the expression of specific microRNA targets. To down-regulate endogenous miRNA levels scientists developed different systems including short hairpin RNA (shRNA) ³³, linear miRNA-sponges ³⁴, circular-RNA miRNA sponges ³⁵, miRNA-targeting Tough Decoy (TuD) RNAs ^{36,37}, miRZips ³⁸, and anti-miRNA antisense oligonucleotides (ASOs) ³⁹, Locked Nucleic Acid (LNA) modified ASOs (LNA-ASOs) to improve DNA nucleases resistance⁴⁰.

ASOs, modified ASOs (LNA-ASOs), and miRZips are synthetic molecules that cannot be expressed in the target cells using genetic systems such as plasmids or viral vectors. As a result, they can only be delivered as synthetic molecules. In contrast, linear miRNA sponges, circular-RNA miRNA sponges, and miRNA-targeting Tough Decoy (TuD) RNAs can be expressed in the target cells by genetic systems (i.e. plasmids or viral vectors).

In the last years, different carriers for miRNA-based therapies are developed, such as liposomes, lipid nanoparticles (LNPs), extracellular vehicles (EVs), macrovesicles and viral vectors ⁴¹. Liposomes mimic the composition of cell membrane. Liposomes are subdivided into neutral, anionic and cationic types, for example Polyethyleneglycol (PEG) lipids are widely used ⁴². However, they can cause undesired immune responses and toxicity. LNPs are mainly used for in vivo delivery of miRNAs due to their numerous advantages, such as payload preservation of miRNA agents, improvement of target specificity and tissue distribution ⁴³. Finally, viral vectors are used to deliver genetic material. Among viral vectors developed for gene delivery in humans there are adeno-associated viruses (AAVs) ⁴⁴. Currently, Adeno-associated virus (AAV) vectors are the leading platform for the delivery of genetic material for the treatment of a variety of human diseases and represent valuable alternative for the delivery of either miRNA mimics (i.e. pre-miRNA) or inhibitors (i.e. linear miRNA sponges, circular-RNA miRNA sponges, miRNA-targeting Tough Decoy (TuD) RNAs, and shRNA) to the lungs.

Current therapies for patients with IPF appear to be poorly effective and most frequently transient with a low survival rate. Nintedanib (Ofev) and Pirfernidone (Esbriet) are the only two approved treatments for IPF patients. Both drugs, if well-tolerated by patients, allow only to reduce lung progressive fibrotic remodeling, with mild efficacy in reducing severe respiratory events and no effect in promoting lung regeneration. Nowadays, IPF still represents the main cause of lung transplant worldwide.

In 2023, the two most promising antifibrotic molecules for the treatment of IPF (Pamrevlumab and PRM-151) failed large Phase III clinical trials (Zephyrus I NCT03955146; Starscape NCT04594707).

Therefore, there is still the need for a treatment for promoting effective lung regeneration in subjects suffering from lung disease characterized by lung fibrotic scarring.

### SUMMARY OF THE INVENTION

Inventors conducted the first-ever phenotypic cell-based High Throughput Screening (HTS) assay on 2042 human microRNA mimics to assess their efficacy in promoting cellular senescence while blocking ATII to ATI trans-differentiation in primary alveolar mouse epithelial cells. Among the tested microRNAs, hsa-miR-210-3p, hsa-miR-155-5p, and hsamiR-490-3p scored as the most effective in promoting cellular senescence (measured by the number of p21-positive ATI cells) while inhibiting ATII to ATI trans-differentiation (measured by the total number of ATI cells).

Human mirR155-5p and miR-210-3p are both well-described hypoxia-regulated miRNAs ^{45,46} and their expression is known to be up-regulated by HIF1-a. Involvement of both miR-155-5p and miR-210-3p has thus far only been elucidated in lung fibroblasts, and their roles in epithelial cells and lung regeneration remain unexplored ⁴⁷. In particular, miR-155-5p has been described as upregulated upon silica-induced fibrosis and its ablation by anti-sense oligonucleotides (ASOs) has been proven effective in the same model ⁴⁸ and miR-210-3p was shown to promote fibroblast proliferation in hypoxic conditions ⁴⁹. Of note, hsa-miR-155-5p, was shown to target TRF1 an essential gene for telomere maintenance thus, if over-expressed, promoting telomere fragility in breast cancer cells ⁵⁰.

To validate the pathological relevance of selected microRNAs, Inventors confirmed that their endogenous expression levels were upregulated in both ATII and lung fibroblast cells isolated from bleomycin treated mice if compared with same cell types isolated from control mice.

Furthermore, ATII cells isolated from bleomycin-treated mice and transfected with LNAs targeting either mmu-miR-210-3p or mmu-miR-155-5p exhibited an improved level of ATII to ATI trans-differentiation and decreased levels of p21-positive cells compared to ATII cells from healthy control mice.

These data suggest that the selected microRNAs miR-210-3p and miR-155-5p represent promising targets for the development of novel therapies for patients with Idiopathic Pulmonary Fibrosis (IPF). These therapies aim to:
1. Reduce the number of senescent alveolar epithelial cells, thereby eliminating the source of paracrine pro-fibrotic signals;
2. Restore the physiological ATII to ATI trans-differentiation process;
3. Eventually, trigger the lung's endogenous regenerative capacity.

Inventors here propose the use of inhibitors of selected human microRNAs to reduce ATII senescence and push the reprogramming of ATII cells into ATI cells as a novel therapeutical strategy to promote lung regeneration after alveolar injury.

Currently there are no available treatments that specifically promote lung regeneration by reducing ATII senescence and/or boosting ATII to ATI trans-differentiation and the disclosed therapeutical approach is based on the idea of reactivating the endogenous alveolar regenerative capacity rather than targeting fibrosis, which is indeed the clinical manifestation of the impossibility of the organ to regenerate.

The present inventors therefore found that inhibitors of microRNAs: miR-210-3p and miR-155-5p are effective for promoting lung regeneration and for the treatment of lung diseases associated with senescence of alveolar epithelial type II and/or type I cells and/or loss of alveolar epithelial type II to type II trans-differentiation capacity and consequent pathological remodelling of the lung, in particular for preventing and/or treating IPF.

It is therefore an object of the invention an agent for use in the prevention and/or treatment of a lung pathological condition associated with senescence of alveolar epithelial type II and/or type I cells and/or loss of alveolar epithelial type II to type I trans-differentiation capacity and/or with loss of lung regenerative capacity and/or for use in promoting lung regeneration in a subject with a lung pathological condition, said agent being selected from the group consisting of :
- an inhibitor of miR-210-3p;
- an inhibitor of miR-155-5p;
or a combination thereof.

Said miR-210-3p can be murine or human, for example it can be hsa-miR-210-3p or mmumiR-210-3p, preferably it is hsa-miR-210-3p

Said miR-155-5p can be murine or human, for example it can be hsa-miR-155-5p or mmumiR-155-5p, preferably, it is hsa-miR-155-5p.

Preferably, the pathological condition is selected from the group consisting of: interstitial lung disease, chronic obstructive pulmonary disease, post-acute respiratory distress syndrome, and post-acute COVID-19 syndrome. Preferably, said interstitial lung disease is selected from the group consisting of idiopathic pulmonary fibrosis, non-specific interstitial pneumonia, desquamative interstitial pneumonia, respiratory bronchiolitis-ILD, cryptogenic organizing pneumonia, and acute interstitial pneumonia.

Preferably the pathological condition is idiopathic pulmonary fibrosis, chronic obstructive pulmonary diseases, or post-acute respiratory distress syndrome. More preferably, it is idiopathic pulmonary fibrosis.

Preferably, said inhibitor is a nucleic acid molecule preferably selected from: a single-stranded or double-stranded nucleic acid molecule, an siRNA molecule, an shRNA molecule, an antisense oligonucleotide (e.g. an antagomir, a Locked Nucleic Acid (LNA) modified ASOs (LNA-ASOs)), a linear miRNA sponge, a circular RNA miRNa sponge, a miRNA-targeting Tough Decoy (TuD) RNA, and a miRZip, derivatives and mixtures thereof. More preferably it is an antisense oligonucleotide comprising at least a locked nucleic acid (LNA).

Preferably, said inhibitor has sufficient complementarity to miR-210-3p, preferably to hsamiR-210-3p, or to miR-155-5p, preferably to hsa-miR-155-5p, to form a hybrid under physiological conditions. Preferably, it comprises at least 7 nucleotides complementary to at least one sequence selected from SEQ ID NO: 1-4, 7-9 or a variant or a fragment thereof, preferably complementary to at least one sequence selected from SEQ ID NO: 1-4, or a variant or a fragment thereof. More preferably, said inhibitor is complementary to the seed sequence of said miRNAs, said sequence being 5'-UGUGCGU-3' for miR-210-3p and 5'-UAAUGCU-3' for miR-155-5p.

Preferably the inhibitor of miR-210-3p comprises a nucleotide sequence comprising or consisting of the sequence 5'-GCTGTCACACGCACA-3' (SEQ ID NO.11) and/or the inhibitor comprises about 8 to about 30 nucleotides in length.

Preferably, the inhibitor of miR-155-5p comprises a nucleotide sequence comprising or consisting of the sequence 5'- TCACAATTAGCATTA-3' (SEQ ID NO.12) and/or the inhibitor comprises about 8 to about 30 nucleotides in length.

Preferably, the inhibitor comprises at least 1 nucleotide, at least 2 nucleotides, at least 3 nucleotides, at least 4 nucleotides, at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, or at least 20 nucleotides at the 5' and/or at the 3' of said nucleotide sequence.

A nucleic acid comprising or consisting of a sequence of SEQ ID NO. 11 or of SEQ ID NO. 12 is also an object of the invention.

Preferably, the inhibitor of hsa-miR-210-3p has at least 50%, 60%, 70%, 80%, 90%, 95%, 99% or 100% complementarity to a portion of a hsa-miR-210-3p sequence.

Preferably, the inhibitor of hsa-miR-155-5p has at least 50%, 60%, 70%, 80%, 90%, 95%, 99% or 100% complementarity to a portion of a hsa-miR-155-5p sequence.

Preferably, the inhibitor binds to a miRNA comprising a nucleic acid that is at least 50%, 60%, 70%, 80%, 90%, 95%, 99% or 100% identical to any one SEQ ID NOs: 1-4, 7-9

Preferably, the inhibitor comprises at least one modified nucleotide, more preferably the at least one modified nucleotide is a locked nucleic acid (LNA), an unlocked nucleic acid (UNA), an arabino nucleic acid (ABA), a bridged nucleic acid (BNA), and/or a peptide nucleic acid (PNA).

Preferably, the inhibitor comprises a backbone modification, more preferably a phosphorodiamidate morpholino oligomer (PMO) and/or phosphorothioate (PS) modification.

Preferably, the delivery agent comprises a vector, preferably a recombinant expression vector or a viral vector, a micelle, an exosome, a lipidoid, a lipoplex, an extracellular vesicle, a synthetic vesicle, a polymeric compound, a peptide, a protein, a cell, a nanoparticle mimic, a nanotube, a conjugate, nanoparticles, microparticles, a liposome or other biological or synthetic vesicle or material including lipid nanoparticles, polymer-based nanoparticles, polymer-lipid hybrid a nanoparticle, a microparticle, a microsphere, a liposome, a colloidal gold particle, a graphene composite, a cholesterol conjugate, a cyclodextran complex, a polyethylenimine polymer, a lipopolysaccharide, a polypeptide, a polysaccharide, a lipopolysaccharide, a collagen or a pegylation of viral vehicle, or combinations thereof.

It is another object of the invention a vector, preferably a recombinant expression vector, comprising a coding sequence for the agent as defined herein and/or expressing the agent as defined herein, such as an inhibitor of hsa-miR-210-3p or an inhibitor of hsa-miR-155-5p, preferably under the control of a suitable promoter, for use in the treatment and/or prevention of lung pathological conditions associated with senescence of alveolar epithelial type II and/or type I cells and/or loss of alveolar epithelial type II to type I trans-differentiation capacity and/or with loss of lung regenerative capacity and/or for use in promoting lung regeneration in a subject with a lung pathological condition, wherein the pathological condition is preferably selected from the group consisting of interstitial lung diseases, chronic obstructive pulmonary diseases, post-acute respiratory distress syndrome, and post-acute COVID-19 syndrome. Preferably, said interstitial lung disease is selected from the group consisting of idiopathic pulmonary fibrosis, non-specific interstitial pneumonia, desquamative interstitial pneumonia, respiratory bronchiolitis-ILD, cryptogenic organizing pneumonia, and acute interstitial pneumonia.

Preferably, the vector is a viral or non-viral vector, more preferably the viral vector is selected from adeno-associated virus (AAV) vectors of any capsid serotype, either natural or artificial, lentivirus vectors, adenoviral vector, retroviral vectors, alphaviral vectors, vaccinia virus vectors, herpes simplex virus (HSV) vectors, rabies virus vectors, and Sindbis virus vectors. Preferably the adeno-associated virus (AAV) vector is AAV6, AAV6.2 or AAV6.2FF.

In an embodiment the vector, preferably an AAV, comprises a coding sequence for at least one inhibitor of miR-210-3p and/or miR-155-5p, preferably wherein said inhibitor is selected from the group of: a linear miRNA sponge, a circular RNA miRNA sponge, a miRNA-targeting Tough Decoy (TuD) RNA, and a shRNA.

A further object of the invention is a host cell transformed with the vector as defined herein for use in the treatment and/or prevention of lung pathological conditions associated with loss of alveolar epithelial type II to type I trans-differentiation capacity and/or with senescence of alveolar epithelial type II and/or type I cells and/or loss of lung regenerative capacity and/or for use in promoting lung regeneration in a subject with a lung pathological condition, wherein the pathological condition is preferably selected from the group of interstitial lung diseases, chronic obstructive pulmonary diseases, post-acute respiratory distress syndrome, and post-acute COVID-19 syndrome. Preferably, said interstitial lung disease is selected from the group consisting of idiopathic pulmonary fibrosis, non-specific interstitial pneumonia, desquamative interstitial pneumonia, respiratory bronchiolitis-ILD, cryptogenic organizing pneumonia, and acute interstitial pneumonia.

Another object of the invention is a recombinant adeno-associated virus (rAAV) particle comprising a nucleic acid encoding the at least one agent as defined herein, preferably the particle comprises a capsid derived from adeno-associated vectors AAV6, AAV6.2, AAV6.2FF, AAV8, AAV1, AAV2, AAV5, or AAV9, preferably wherein the nucleic acid is operably linked to a viral promoter or a tissue specific promoter for use in the treatment and/or prevention of lung pathological conditions associated with senescence of alveolar epithelial type II and/or type I cells and/or loss of alveolar epithelial type II to type I trans-differentiation capacity and/or with loss of lung regenerative capacity and/or for use in promoting lung regeneration in a subject with a lung pathological condition, wherein the pathological condition is preferably selected from the group of interstitial lung diseases, chronic obstructive pulmonary diseases, post-acute respiratory distress syndrome, and post-acute COVID-19 syndrome. Preferably, said interstitial lung disease is selected from the group consisting of idiopathic pulmonary fibrosis, non-specific interstitial pneumonia, desquamative interstitial pneumonia, respiratory bronchiolitis-ILD, cryptogenic organizing pneumonia, and acute interstitial pneumonia.

It is an object of the present invention a pharmaceutical composition for use in the treatment and/or prevention of lung pathological conditions associated with loss of alveolar epithelial type II to type I trans-differentiation capacity and/or with senescence of alveolar epithelial type II and/or type I cells and/or loss of lung regenerative capacity and/or for use in promoting lung regeneration in a subject with a lung pathological condition, wherein the pathological condition is preferably selected from the group consisting of interstitial lung diseases, chronic obstructive pulmonary diseases, post-acute respiratory distress syndrome, and post-acute COVID-19 syndrome, said pharmaceutical composition comprising an agent as defined herein, or the nucleic acid as defined herein, or the vector as defined herein, or the host cell as defined herein, or a recombinant adeno-associated virus (rAAV) particle as defined herein and at least one pharmaceutically acceptable vehicle and/or excipient. Preferably, said interstitial lung disease is selected from the group consisting of idiopathic pulmonary fibrosis, non-specific interstitial pneumonia, desquamative interstitial pneumonia, respiratory bronchiolitis-ILD, cryptogenic organizing pneumonia, and acute interstitial pneumonia.

### DETAILED DESCRIPTION OF THE INVENTION

Inventors performed a cell based HTS assay on 2042 human microRNAs for their efficacy in promoting ATII to ATI trans-differentiation in primary alveolar mouse epithelial cells (ex vivo). They identified three microRNA mimics that respectively scored as the most effective in promoting ATII cellular senescence while inhibiting ATII to ATI trans-differentiation. Two out of three microRNAs, specifically hsa-miR-155-5p and hsa-miRNA-210-3p, were endogenously expressed by both mouse and human ATII cells. The levels of selected microRNAs were proved to be upregulated in the lungs of mice treated with Bleomycin, a chemical agent known to promote pulmonary fibrosis and therefore inducing ATII cellular senescence and apoptosis. To characterize the effect of LNAs targeting the microRNAs, mmumiR-155-5p and mmu-miRNA-210-3p, inventors isolated diseased ATII cells from bleomycin-treated mice lungs and transfected them with selected LNAs.

Inventors demonstrated that LNAs targeting mmu-miR-155-5p and mmu-miRNA-210-3p, completely rescued ATII to ATI trans-differentiation, in diseased mouse ATII cells, if compared to untreated controls.

The agents of the invention are able to reduce ATII senescence and/or reprogram ATII into ATI and/or to promote and/or increase ATII to ATI trans-differentiation.

The sequence of hsa-miR-155-5p is available in the state of the art with the ID hsa-miR-155-5p (www.mirbase.org; Database Release 21; stem-loop sequence accession number MI0000681, mature sequence accession number MIMAT0000646).

The sequence of hsa-miR-210-3p is available in the state of the art with the ID hsa-miR-210-3p (www.mirbase.org; Database Release 21; stem-loop sequence accession number MI0000286, mature sequence accession number MIMAT0000267).

In particular, hsa-miR-155-5p can have anyone of the following sequences: 
5’-
CUGUUAAUGCUAAUCGUGAUAGGGGUUUUUGCCUCCAACUGACUCCUACAUA
UUAGCAUUAACAG-3’ (SEQ ID NO. 2)
corresponding to the accession number MI0000681;
5'-UUAAUGCUAAUCGUGAUAGGGGU-3' (SEQ ID NO. 1)
   corresponding to the mature sequence accession number MIMAT0000646.

In particular, hsa-miR-210-3p can have anyone of the following sequences: 
5’-
ACCCGGCAGUGCCUCCAGGCGCAGGGCAGCCCCUGCCCACCGCACACUGCGCU
GCCCCAGACCCACUGUGCGUGUGACAGCGGCUGAUCUGUGCCUGGGCAGCGCG
ACCC-3’ (SEQ ID NO. 4)
corresponding to the accession number MI0000286;
5'-CUGUGCGUGUGACAGCGGCUGA-3' (SEQ ID NO. 3)
   corresponding to the mature sequence accession number MIMAT0000267.

In some embodiment, the inhibitor for the use of the invention may also be complementary, at least partially, to one of the following sequences: 
5’-
CUGUUAAUGCUAAUUGUGAUAGGGGUUUUGGCCUCUGACUGACUCCUACCUG
UUAGCAUUAACAG-3’ (SEQ ID NO. 8)
corresponding to the accession number MI0000177 of mmu-miR-155-5p;
5'- UUAAUGCUAAUUGUGAUAGGGGU-3' (SEQ ID NO. 7)
   corresponding to the mature accession number MIMAT0000165 of mmu-miR-155-5p; 
5’-
CCGGGGCAGUCCCUCCAGGCUCAGGACAGCCACUGCCCACCGCACACUGCGU
UGCUCCGGACCCACUGUGCGUGUGACAGCGGCUGAUCUGUCCCUGGGCAGC
GCGAACC-3’ (SEQ ID NO. 9)
corresponding to the accession number MI0000695 of mmu-miR-210-3p.

Inhibitors of anyone of the above sequences can be used according to the present invention. Also, inhibitors of any functional fragment of any of the above sequences can be used according to the present invention.

Preferably, in the present invention:
- hsa-miR-155-5p comprises or consists of the sequence with SEQ ID NO: 1
- hsa-miR-210-3p comprises or consists of the sequence with SEQ ID NO: 3
- mmu-miR-210-3p comprises or consists of the sequence with SEQ ID NO: 3
- mmu-miR-155-5p comprises or consists of the sequence with SEQ ID NO: 7.

The hsa-miRs or miRs or miRNAs or mmu-miRs mentioned in the present invention preferably comprise or consist of the herein indicated sequences. They also may comprise homologs, analogs and orthologues thereof, primary miRNA molecules, precursor miRNA molecules, mature miRNA molecules, and miRNA mimetics. The nucleic acids may be selected from RNA, DNA or nucleic acid analog molecules, e.g. as sugar- or backbone-modified ribonucleotides or deoxyribonucleotides, peptide nucleic acids (PNA) or locked nucleic acids (LNA).

In the present invention any combination of the above agents may be used. E.g. inhibitors of miR-210-3p and miR-155-5p can be used together. More than one agent may be used.

A "miRNA inhibitor" or "inhibitor of" a miR as used herein, refers to a compound that can decrease, alter, and/or modulate miRNA expression, function, and/or activity.

The miRNA inhibitor can be a polynucleotide sequence that is at least partially complementary to the target miRNA nucleic acid sequence, such that the miRNA inhibitor hybridizes to the target miRNA sequence. For instance, in some aspects, a miRNA inhibitor of the present disclosure comprises a nucleotide sequence encoding a nucleotide molecule that is at least partially complementary to the target miR nucleic acid sequence, such that the miRNA inhibitor hybridizes to the miR sequence. In further aspects, the hybridization of the miRNA inhibitor to the miR sequence decreases, alters, and/or modulates the expression, function, and/or activity of the miR. "miRNA inhibitor" may refer to a nucleotide sequence that is a reverse complement of a mature miRNA (the target site). In some embodiments, the miRNA inhibitor is chemically synthesized and/or is chemically modified to prevent nucleic acidprotein complex-induced cleavage (e.g., RISC-induced cleavage) of miRNA, enhance binding affinity to the target site, and/or provide resistance to nucleolytic degradation of the miRNA inhibitor. As used herein, a miRNA inhibitor includes any natural or artificial RNA transcripts that sequestrate miRNAs and decrease or eliminate their effects. Included herein are miRNA inhibitors that are identical to competing endogenous RNAs (ceRNAs). ceRNAs are natural and intracellular miRNA inhibitors that compete to bind to shared miRNA recognition elements (MREs) to decrease microRNA availability and relieve the repression of target RNAs.

In some embodiments, the inhibitor comprises or consist of a nucleic acid at least partially complementary to any one of SEQ ID NOs: 1-4, 7-9, preferably at least partially complementary to any one of SEQ ID NOs: 1-4, more preferably at least partially complementary to any one of SEQ ID NOs: 1 or 3.

In some embodiments, the nucleic acid sequence that is complementary to a target miRNA miR-210-3p and/or miR-155-5p can be an interfering RNA, including but not limited to a small interfering RNA ("siRNA") or a small hairpin RNA ("shRNA"). Methods for constructing interfering RNAs are well known in the art. For example, the interfering RNA can be assembled from two separate oligonucleotides, where one strand is the sense strand and the other is the antisense strand, wherein the antisense and sense strands are self-complementary (i.e., each strand comprises nucleotide sequence that is complementary to nucleotide sequence in the other strand; such as where the antisense strand and sense strand form a duplex or double stranded structure); the antisense strand comprises nucleotide sequence that is complementary to a nucleotide sequence in a target nucleic acid molecule or a portion thereof (i.e., an undesired gene) and the sense strand comprises nucleotide sequence corresponding to the target nucleic acid sequence or a portion thereof. Alternatively, interfering RNA is assembled from a single oligonucleotide, where the self-complementary sense and antisense regions are linked by means of nucleic acid based or non-nucleic acid-based linker(s). The interfering RNA can be a polynucleotide with a duplex, asymmetric duplex, hairpin or asymmetric hairpin secondary structure, having self-complementary sense and antisense regions, wherein the antisense region comprises a nucleotide sequence that is complementary to nucleotide sequence in a separate target nucleic acid molecule or a portion thereof and the sense region having nucleotide sequence corresponding to the target nucleic acid sequence or a portion thereof. The interfering can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siRNA molecule capable of mediating RNA interference.

In some embodiments, the interfering RNA coding region encodes a self-complementary RNA molecule having a sense region, an antisense region and a loop region. Such an RNA molecule, when expressed desirably forms a "hairpin" structure and is referred to herein as an "shRNA." The loop region is generally between about 2 and about 10 nucleotides in length. In some embodiments, the loop region is from about 6 to about 9 nucleotides in length. In some embodiments, the sense region and the antisense region are between about 15 and about 20 nucleotides in length. Following post-transcriptional processing, the small hairpin RNA is converted into a siRNA by a cleavage event mediated by the enzyme Dicer, which is a member of the RNase III family. The siRNA is then capable of inhibiting the expression of a gene with which it shares homology. For details, see ^{33 51 52 53 54 55 56 57}.

In some embodiments, the inhibitor can be a linear miRNA sponge, a circular RNA miRNa sponge, a miRNA-targeting Tough Decoy (TuD) RNA, miRZip or nucleic acids coding thereof. MiRNA linear sponges or decoys ^{58 34} are in vivo expressed transcripts that contain multiple high affinity miRNA antisense binding sites (MBS) that mimic those found in mRNAs and complementary to a miRNA to be targeted; these transcripts can efficiently sequester specific miRNAs and, thereby, prevent their binding to endogenous target genes. These binding sites are usually tandem repeats of identical sites designed to target either single specific miRNAs or miRNA family members sharing the same seed region; considering that the interaction between microRNA and target is dependent on base-pairing in the seed region (positions 2-8 of the microRNA), a decoy target should interact with all members of a microRNA seed family. Design of miRNA sponges or decoys have been disclosed for instance in Ebert et al. ³⁴ and Kluiver et al ⁵⁸. Sponge sequences typically consist of multiple miRNA binding sites (MBS) separated by 4-6 nucleotides-long spacer sequences. MBSs are either perfectly anti-sense or contain a bulge at the central position. MiRNA sponges offer an advantage of inhibiting all seed family members and also when multiple MBS are introduced, they can be used for inhibition of whole miRNA clusters. MiRNA sponge construct design and off-targets testing may be performed using a web-based tool MiRNAsong: microRNA Sponge Generator and tester. Suitably, a Sponge is composed of a high affinity miRNA antisense binding site (MBS) which includes a sequence complementary to the target miRNA, and a "bulge" region, i.e. a mismatched region in nucleotides 9 to 12 of the MBS sequence. Suitably, the complementary region is at least 75 %, 80 %, 85 % , 90%, 95%, 99% or 100% identical to the target miRNA miR-210-3p and/or miR-155-5p. Most preferably, the complementary region is at least 80% identical to the target miRNA. Suitably, sequence complementary to the target miRNA sequences, miR-210-3p and/or miR-155-5p, includes a sequence complementary to the SEED sequence, e.g. the sequence within a given miRNA which is essential to the target transcript recognition, and the "bulge" region is outside said Seed sequence. Said Seed sequence preferably comprises or consists of the sequence 5'-UGUGCGU-3' for miR-210-3p and of the sequence 5'-UAAUGCU-3' for miR-155-5p. Each MBS sequence (MBS SEQ) is the same length as the mature target miRNA. Each MBS SEQ is repeated several times, between 6 and 24 times. Suitably, MBS SEQs are separated from each other by a "spacer" sequence consisting of 4 to 6 nucleotides.

In some embodiments, the inhibitor can be a circular RNA miRNA sponge. A circular RNA miRNA sponge is a RNA sponge with a peculiar closed-loop structure that helps in preventing its degradation, thus increasing stability and sponging efficacy over time. Circular RNAs (circRNAs) are covalently closed-loop ssRNA molecules that lack 5-caps and 3-poly(A) tails ⁵⁹. CircRNAs are generated by back-splicing, a process in which the 3'-end splice site of a precursor mRNAs is linked with the upstream 5'-end splice site to form a closed-loop ⁵⁹. This closed-loop structure makes circRNAs resistant to RNA exonucleases-mediated degradation ⁵⁹. CircRNAs are naturally occurring in mammalian cells and can act as microRNA sponges by comprising within their sequence multiple miRNA target binding sites, thus negatively regulating microRNA activity by competing with mRNA-microRNA binding. CircCD44 (anti miR-502), cIRS-7 (anti miR-7), and circLRP6 (anti miR-145) are some examples ⁵⁹. CircRNAs can be invitro engineered to generate artificial circRNA sponges to specifically therapeutically target one or more microRNAs; such moelcules are generally termed as circmiRs. In example, Lavenniah et al. generated a circmiR targeting miR-132 and miR-212, two known cardiac prohypertrohpic microRNA; such circmiR has been delivered in vivo to cardiac myocytes by recombinant AAV vector and therapeutically reduced miR-132 and miR-212 level as well as cardiac hypertrophic remodeling ³⁵. The sequence design of circmiRs, depends on the number of miRNAs to be targeted and it relies on the same parameters considered for linear miRNA sponges, as discussed above. In particular, a circular RNA miRNA sponge for use according to the invention may comprise one or more sequences complementary to the target miRNA sequences, i.e. miR-210-3p and/or miR-155-5p, preferably including at least one sequence complementary to the seed sequences of such miRNAs, e.g. 5'-UGUGCGU-3' for miR-210-3p and 5'-UAAUGCU-3' for miR-155-5p.

In some embodiments, the inhibitor can be a miRNA-targeting Tough Decoy (TuD) RNA. Tough Decoy (TuD) RNA inhibitors are synthetic, not naturally occurring, ssRNA DNA-encoded miRNA inhibitors first reported by Haragushi et al ³⁶ and they consist of a hairpin containing a large, internal bulge exposing two miRNA target sites with imperfect base-pairing with the target miRNA sequence ^{37 36}. Compared to linear sponge RNAs, Tough Decoy (TuD) RNA inhibitors, being shorter, were proven to be less sensitive to targeting by endogenous miRNAs and, therefore, less prone to down regulation than sponge-encoding vectors ⁶⁰. Tough Decoy (TuD) RNA inhibitors, were successfully delivered in vivo by rAAV vectors. In particular, rAAV9 expressing anti-miR-122 or anti-let-7 TuDs were successfully used to reduce target miRNA levels in the liver by promoting miRNA tailing and trimming ⁶¹; similarly, AAV9-miR-25 TuD delivered to the murine pressure-overload heart failure model selectively decreased expression of miR-25 in cardiomyocytes, increased levels of SERCA2a protein, and ameliorated cardiac dysfunction and fibrosis ⁶². A miRNA-targeting Tough Decoy (TuD) RNA targeting miR-210-3p and/or miR-155-5p can be manufactured according to common general knowledge in the field, such as above cited references.

In some embodiments, the inhibitor can be a miRNA zipper (miRZip). MiRZip are synthetic DNA molecules, not naturally occurring, and specifically designed to bind to the 5'-half sequence of one molecule and the 3'-half sequence of another molecule of the target miRNA through a complementary interaction, thus connecting the target miRNA molecules end-to-end, and therefore blocking the functions of targeted miRNA. MiRZip usually includes LNA modified bases to increase stability and specificity. As reported by Meng et al, miRZips targeting two miRNAs, miR-221 and miR-17, have been validated in human breast cancer cell lines demonstrating 70% to 90% knockdown of miRNA levels by corresponding small RNA zippers at 24 h after transfection ³⁸. A miRzip targeting miR-210-3p and/or miR-155-5p can be manufactured according to common general knowledge in the field, such as above cited reference.

The terms "miRNA", "miR" and "microRNA" are used interchangeably and refer to a microRNA molecule found in eukaryotes that is involved in RNA-based gene regulation. Such terms refer to the single-stranded RNA molecule processed from a precursor. In some aspects, the term "antisense oligomers" can also be used to describe the microRNA molecules of the present disclosure. MicroRNAs recognize and bind to target mRNAs through imperfect base pairing leading to destabilization or translational inhibition of the target mRNA and thereby downregulate target gene expression. Conversely, targeting miRNAs via molecules comprising a miRNA binding site (generally a molecule comprising a sequence complementary to the seed region of the miRNA) can reduce or inhibit the miRNA-induced translational inhibition leading to an upregulation of the target gene.

In some aspects, a miR inhibitor of the present invention comprises a nucleotide sequence encoding a nucleotide molecule that comprises at least one miR binding site, wherein the nucleotide molecule does not encode a protein. In some aspects, the miR binding site is at least partially complementary to the target miRNA nucleic acid sequence, such that the miR inhibitor hybridizes to the miR nucleic acid sequence. In some aspects, the miR binding site of a miR inhibitor disclosed herein has at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, at least about 99%, or about 100% sequence complementarity to the nucleic acid sequence of the miR. In certain aspects, the miR binding site is fully complementary to the nucleic acid sequence of the miR.

In certain aspects, the miR binding site is complementary to the miR except for 1, 2, 3, 4, 5, 6, 7, 8, 9, or 10 mismatches.

MiRNA sequences and miRNA binding sequences that can be used in the context of the present invention include, but are not limited to, all or a portion of the sequences herein disclosed, as well as the miRNA precursor sequence, or complement of one or more of these miRNAs. Any aspects of the disclosure involving specific miRNA binding sites by name is contemplated also to cover miRNAs complementary sequences whose sequences are at least about 50%, at least about 55%, at least about 60%, at least about 65%, at least about 70%, at least about 71%, at least about 72%, at least about 73%, at least about 74%, at least about 75%, at least about 76%, at least about 77%, at least about 78%, at least about 79%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or about 100% identical to the mature sequence of the specified miRNA complementary sequence.

In some embodiments, the miRNA inhibitor binds to a miRNA comprising a nucleic acid that has at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, least 81%, least 82%, least 83%, least 84%, least 85%, least 86%, least 87%, least 88%, least 89%, at least 90%, at least 91%, at least 92%, at least 93%, at least 94%, at least 95%, at least 96%, at least 97%, at least 98%, at least 99% sequence identity, or at least 100% sequence identity ( e.g ., at least 99.5% sequence identity, at least 99.6% sequence identity, at least 99.7% sequence identity, at least 99.8% sequence identity, at least 99.9% sequence identity, or at least 100% sequence identity) to the nucleic acid sequence of any one of SEQ ID NOs. 1-4, 7-9. In some embodiments, the miRNA inhibitor binds to a miRNA comprising a nucleic acid that is any one of SEQ ID NOs. 1-4, 7-9, preferably SEQ ID NOs. 1-4.

In some embodiments, the miRNA inhibitors are for example at least 5, 10, 20, 25, 30, 35, 40, 45, 50, 55, 60, 65, 70, 75, 80, 85, 90, 95, 100, 105, 110, 115, or 120 nucleotides in length. In some embodiments, the miRNA inhibitors are no more than 600, 500, 400, 300, 200, 120, 115, 110, 100, 95, 90, 85, 80, 75, 70, 65, 60, 55, 50, 45, 40, 35, 30, 25, 20, 10, or 5 nucleotides in length. In some embodiments, the miRNA inhibitors are at least 13 nucleotides in length. In some embodiments, the miRNA inhibitors are no more than 22 nucleotides in length. In some preferred embodiments, the miRNA inhibitors are 13 to 16 nucleotides in length. A miRNA inhibitor disclosed herein may comprise at least 1, 2, 3, 4, 5, 6, 7, 8, 9, 10, 11, 12, 13, 14, 15, 16, 17, 18, 19, or 20 substitutions.

A miRNA inhibitor is preferably between about 7 to 35 nucleotides (e.g., 17 to 25 nucleotides) in length and comprises a 5' to 3' sequence that is at least 90% complementary to the 5' to 3' sequence of a mature miRNA. In certain embodiments, a miRNA inhibitor molecule is 17, 18, 19, 20, 21, 22, 23, 24, or 25 nucleotides in length, or any range derivable therein. Moreover, an miRNA inhibitor has a sequence (from 5' to 3') that is or is at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 90, 91, 92, 93, 94, 95, 96, 97, 98, 99, 99.1, 99.2, 99.3, 99.4, 99.5, 99.6, 99.7, 99.8, 99.9 or 100% complementary, or any range derivable therein, to the 5' to 3' sequence of a mature miRNA, particularly a mature, naturally occurring miRNA. One of skill in the art could use that portion of the longer probe sequence that is has at least partial complementarity to at least a portion of a sequence of a mature miRNA as the sequence for a miRNA inhibitor.

In certain embodiments, a vector comprises the miRNA inhibitor as described herein.

In certain embodiments, the miRNA inhibitors or agent provided herein comprise one or more chemical modifications, wherein the modification facilitates the penetration of a cellular membrane in the absence of a delivery vehicle. Any chemical modification of the miRNA inhibitor that sustains the same functional structure will bind with its intended target. Examples of any of the chemical modifications include a 2'-O-methylated nucleoside (2OMe), a 2'-fluoro oligonucleotide (2'F), a 2'-0-methoxy ethyl oligonucleotide (2'MOE), a N6-methyladenosine (m6A), a phosphorodiamidate morpholino oligonucleotide (PMO), a peptide nucleic acid (PNA), a phosphorothioate bond (PS), a locked nucleic acid (LNA), a non-nucleotide N,N-diethyl-4-(4-nitronaphthalen-1-ylazo)-phenylamine (ZEN), a hydrophobic moiety, a naphthyl modifier, or a cholesterol moiety. In certain embodiments, the chemical modification of the miRNA is produced by methylation.

The miRNA inhibitors or agent described herein can employ a variety of oligonucleotide chemistries. Examples of oligonucleotide chemistries include, without limitation, peptide nucleic acid (PNA), locked nucleic acid (LNA), phosphorothioate, 2'0-Me-modified oligonucleotides, and morpholino chemistries, including combinations of any of the foregoing. The structures of LNAs can be found, for example, in Wengel, et al., Chemical Communications (1998) 455; Tetrahedron (1998) 54:3607, and Accounts of Chem. Research (1999) 32:301); Obika, et al., Tetrahedron Letters (1997) 38:8735; (1998) 39:5401, and Bioorganic Medicinal Chemistry (2008) 16:9230. Agents provided herein may incorporate one or more LNAs; in some cases, the compounds may be entirely composed of LNAs.

In an embodiment, the inhibitor comprises a nucleotide sequence comprising or consisting of the sequence of SEQ ID NO.11 wherein at least one nucleotide is a LNA. In some embodiments, said nucleotide sequence comprises 1, 2, 3, 4, 5, 6, 7, 8, 9, 10, 11, 12, 13, 14 or 15 LNAs.

In an embodiment, the inhibitor comprises a nucleotide sequence comprising or consisting of the sequence of SEQ ID NO.12 wherein at least one nucleotide is a LNA. In some embodiments, said nucleotide sequence comprises 1, 2, 3, 4, 5, 6, 7, 8, 9, 10, 11, 12, 13, 14 or 15 LNAs.

The miRNA inhibitors or agent described herein can be prepared by any appropriate method known in the art. For example, in some embodiments, the miRNA inhibitors described herein are prepared by chemical synthesis or in vitro transcription.

In the present methods, miRNA inhibitor or agent described herein can be administered to the subject, for example, as nucleic acid without a delivery vehicle, in combination with a delivery reagent, and/or as a nucleic acid comprising sequences that express the miRNA inhibitor or agent described herein. In some embodiments, any nucleic acid delivery method known in the art can be used in the methods described herein.

The microRNAs of the invention have been identified for the claimed use through a highcontent, fluorescence-microscopy-based, high-throughput screen performed in primary mouse alveolar type II epithelial cells (ATII) cells using a library of 2042 miRNA mimics.

The use of nucleic acid or DNA coding for the inhibitor as defined above is also within the scope of the present invention. Such DNA, for example a cDNA, can be designed according to the general knowledge in the field.

Also included in the present invention are inhibitors of variants of mature miRNAs and of precursor miRNAs of the invention comprising a sequence with at least 50%, 60%, 70%, 75%, 80%, 85%, 90%, 95% identity to the reference sequences or DNA molecules encoding said miRNAs.

It has been found that hsa-miR-210-3p and hsa-miR-155-5p scored as the most effective in increasing cellular senescence and blocking trans-differentiation of ATII cells into ATI cells with a Z-Score (a statistic parameter showing how a treatment is far from the mean of all treatments) ≥1.65 at 72 hours post transfection.

Therefore, the above microRNAs are able to effectively inhibit the trans-differentiation of primary murine ATII cells into ATI cells by promoting cellular senescence.

Therefore, inhibitors of said miRNAs can be used for the treatment of all lung diseases in which the senescence of ATII cells or loss of alveolar epithelial type II to type I trans-differentiation capacity is the driver of the disease (for example, ILDs, IPF, chronic obstructive pulmonary (or lung) disease, post-acute respiratory distress syndrome). The present agents can therefore be used for medical use, in particular for the treatment of pathological conditions associated loss of alveolar epithelial type II to type I trans-differentiation capacity and/or with senescence of alveolar epithelial type II and/or type I cells and/or loss of lung regenerative capacity and/or for promoting lung regeneration, wherein the pathological condition is preferably selected from the group of interstitial lung diseases, chronic obstructive pulmonary diseases, post-acute respiratory distress syndrome, and post-acute COVID-19 syndrome, said interstitial lung disease being preferably selected from idiopathic pulmonary fibrosis, non-specific interstitial pneumonia, desquamative interstitial pneumonia, respiratory bronchiolitis-ILD, cryptogenic organizing pneumonia, and acute interstitial pneumonia.

The present agents can therefore be used for the treatment of interstitial lung diseases, chronic obstructive pulmonary diseases, post-acute respiratory distress syndrome, and post-acute COVID-19 syndrome, said interstitial lung disease being preferably selected from idiopathic pulmonary fibrosis, non-specific interstitial pneumonia, desquamative interstitial pneumonia, respiratory bronchiolitis-ILD, cryptogenic organizing pneumonia, and acute interstitial pneumonia.

In an embodiment, an agent herein described is used for the treatment of a lung disease associated with senescence of alveolar epithelial type II and/or type I cells and/or loss of alveolar epithelial type II to type I trans-differentiation capacity and/or with loss of lung regenerative capacity and/or for promoting lung regeneration in a subject with a lung disease, when the acute phase of the inflammatory response of said disease is ceased. For "acute phase of the inflammatory response of the disease" is intended the phase of the disease characterized by an acute lung inflammatory response, typically immediately followed by an alveolar damage. According to this embodiment, the agents herein described can be advantageously administered to a subject affected by a lung disease in order to promote lung regeneration independently of both the type of insult and the state of inflammatory response.

For "promoting lung regeneration" it is herein intended promoting and/or increasing and/or reactivating the endogenous alveolar regenerative capacity of the lung. In particular, it is intended promoting and/or increasing ATIIs to ATIs trans-differentiation in the lung. In a particular embodiment, lung regeneration is promoted after an alveolar injury.

For "senescence of alveolar epithelial type II and/or type I cells" is intended the permanent arrest of cell cycle, the acquisition of characteristic senescence markers (i.e. p21 positivity), the loss of normal cellular functions, the abnormal activation of multiple pathways, the acquisition of secretory associated senescent phenotype (SASP), and/or the shortening or damage of telomeres.

The skilled person in the field, for example a physician specialized in the lung disease, is able to identify the pathologies characterized by pathological remodelling of lung architecture, fibrosis, loss of alveoli, and eventually reduced gas-exchange capacity, according to the common knowledge in the field, which may characterize the above indicated conditions.

Said inhibitors or agents can also be advantageously used in any condition at risk of developing lung diseases as defined above.

Indeed, for prevention it is intended the administration of the microRNA inhibitor or agent to a subject liable or at risk to develop a condition herein mentioned due, for example, to genetic predisposition (e.g. familial Pulmonary fibrosis), environment conditions (e.g. organic dust (livestock/agriculture/farming), metal and mineral dust, wood dust, asbestos, cigarette smoking) or the presence of some conditions or pathologies eventually leading to any pathological conditions associated with loss of alveolar epithelial type II to type I transdifferentiation capacity and/or with loss of lung regenerative capacity and/or for use in promoting lung regeneration, wherein the pathological condition is preferably selected from the group of interstitial lung diseases, chronic obstructive pulmonary diseases, post-acute respiratory distress syndrome, and post-acute COVID-19 syndrome, said interstitial lung disease being preferably selected from idiopathic pulmonary fibrosis, non-specific interstitial pneumonia, desquamative interstitial pneumonia, respiratory bronchiolitis-ILD, cryptogenic organizing pneumonia, and acute interstitial pneumonia.

The use of the present inhibitors in gene therapy is also a preferred embodiment of the invention.

For gene therapy it is intended the therapeutic delivery of nucleic acids into a patient's cells as a drug to treat a disease. According to the present invention, the agent of the invention can be delivered to cells of a subject in need thereof, for example a subject affected by or at risk of developing any of the above-mentioned diseases, in order to treat such diseases.

A further object of the present invention is an RNA stretch comprising the said microRNA inhibitor. The notion of RNA stretch as a continuous tract of RNA is commonly known in the field. Said RNA stretch can be obtained in vitro through cell-free transcription methods, produced synthetically or expressed in the cells upon transfer of the relative DNA coding sequence, or introduced or expressed in the cells by administration of a plasmid, a viral or other type of vector.

The present invention provides said RNA stretch for use as a medicament for prevention and/or treatment of any of the above-mentioned diseases.

In embodiments of the present invention, the agent of the invention can be administered to a subject as a medicament by conventional methods.

Conveniently, said medicament or agent or pharmaceutical composition as defined herein is in the form of a preparation for intra-tracheal instillation, inhalation (Aerosol), parenteral administration, intravenous administration, but other forms are equally suitable for carrying out the present invention. The person skilled in the art will decide the effective time of administration, depending on the patient's conditions, degree of severity of the disease, response of the patient and any other clinical parameter within the general knowledge of this matter.

Another object of the present invention is a pharmaceutical composition comprising the above defined agent as active ingredient for the prevention and/or treatment of any of the above-mentioned diseases.

The pharmaceutical composition according to the invention may contain at least one of the following: a synthetic DNA coding for to an inhibitor of the microRNA herein described, or of its primary transcript or precursor, a synthetic RNA corresponding to said inhibitor.

The agent of the present invention or the corresponding coding DNA can be administered naked or together with lipid nanoparticle or lipidic molecules such as cationic lipids, or peptides, or in the context of polymeric scaffolds, which can facilitate their delivery, according to the art.

When the agent is administered using as a vector a lipidic molecule or nanoparticle, such as a liposome, the miRNA inhibitor is preferably binding the sequence corresponding to SEQ ID NO: 1, 3 and 7 (corresponding to mature miRNA sequences).

Another method to administer such agent or its corresponding DNA is by means of a suitable vector known for the administration of RNA or DNA.

A preferred vector is the adeno-associated vector (AAV) of any capsid serotype, either natural (such as, but not restricted to, AAV6, AAV1, AAV2, AAV8 or AAV9) or artificial (AAV6.2, AAV6.2FF ^{63,64}.

When the agent is administered using as a vector a viral vector, the miRNA inhibitor is preferably binding the sequence corresponding to SEQ ID NO: 1, 2, 3, 4, 7, 8 or 9.

The vector can be designed according to common general knowledge in the field. Typically, it comprises a suitable promoter, such as a CMV promoter or a U6 promoter, and a cDNA sequence coding for an agent as described herein.

A vector for use according to the present invention comprising at least the agent as defined above and/or a DNA coding for at least said agent, or a combination thereof is also an object of the invention.

All these methods and formulations to administer a microRNA inhibitor corresponding to the agent of the present invention, DNA coding for said agent, are conventional and well known in the art and do not need further explanations.

In particular, the skilled person knows how to choose the suitable administration mode and vector, for example for human administration, according to the general knowledge in the field.

Injection is a preferred administration route. However, the skilled person in the art can decide to administer the agents by means of any conventional pharmaceutical composition. Reference can be made to Remington's Pharmaceutical Sciences, last edition.

The administration regime, dosage and posology will be determined by the physician according to his experience, the disease to be treated and the patient's conditions.

According to the administration route chosen, the compositions will be in solid, liquid or nebulized form, suitable for oral, parenteral, intravenous or intra-tracheal administration.

The compositions according to the present invention contain, along with the agent, at least one pharmaceutically acceptable vehicle or excipient. These may be particularly useful formulation co-adjuvants, e.g. solubilising agents, dispersing agents, suspension agents, and emulsifying agents.

Average quantities of the active agent may vary and in particular should be based upon the recommendations and prescription of a qualified physician.

The present invention also refers to the herein defined agents, pharmaceutical compositions, nucleic acids, vectors, host cells, recombinant adeno-associated virus (rAAV) particles and also refers to their medical use.

The present invention also refers to the herein defined agents, pharmaceutical compositions, nucleic acids, vectors, host cells, recombinant adeno-associated virus (rAAV) particles for use in the prevention and/or treatment of an interstitial lung disease.

The present invention also refers to the herein defined agents, pharmaceutical compositions, nucleic acids, vectors, host cells, recombinant adeno-associated virus (rAAV) particles for use in the prevention and/or treatment of idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases or post-acute respiratory distress syndrome or post-acute COVID-19 syndrome.

In the context of the present invention, the lung pathological condition associated with senescence of alveolar epithelial type II and/or type I cells and/or loss of alveolar epithelial type II to type I trans-differentiation capacity and/or with loss of lung regenerative capacity may be an interstitial lung disease.

In the context of the present invention, the lung pathological condition associated with senescence of alveolar epithelial type II and/or type I cells and/or loss of alveolar epithelial type II to type I trans-differentiation capacity and/or with loss of lung regenerative capacity may be idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases or post-acute respiratory distress syndrome or post-acute COVID-19 syndrome.

In the context of the present invention, the lung pathological condition associated with senescence of alveolar epithelial type II and/or type I cells and/or loss of alveolar epithelial type II to type I trans-differentiation capacity and/or with loss of lung regenerative capacity may be idiopathic pulmonary fibrosis, chronic obstructive pulmonary diseases or post-acute respiratory distress syndrome. Preferably, it is idiopathic pulmonary fibrosis.

In the context of the present invention, the lung pathological condition may be an interstitial lung disease.

In the context of the present invention, the lung pathological condition may be idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases or post-acute respiratory distress syndrome or post-acute COVID-19 syndrome.

In the context of the present invention, the lung pathological condition may be idiopathic pulmonary fibrosis, chronic obstructive pulmonary diseases or post-acute respiratory distress syndrome. Preferably, it is idiopathic pulmonary fibrosis.

In the context of the present invention, the interstitial lung disease is preferably idiopathic pulmonary fibrosis, non-specific interstitial pneumonia, desquamative interstitial pneumonia, respiratory bronchiolitis-ILD, cryptogenic organizing pneumonia or acute interstitial pneumonia.

It is also an object of the present invention a method of prevention and/or treatment of lung pathological conditions associated with senescence of alveolar epithelial type II and/or type I cells and/or loss of alveolar epithelial type II to type I trans-differentiation capacity and/or with loss of lung regenerative capacity, comprising administering at least one agent, pharmaceutical composition, nucleic acid, vector, host cell, recombinant adeno-associated virus (rAAV) particle or combination thereof as defined herein to a patient in need thereof.

It is also an object of the present invention a method of promoting lung regeneration in a subject with a lung pathological condition, comprising administering at least one agent, pharmaceutical composition, nucleic acid, vector, host cell, recombinant adeno-associated virus (rAAV) particle or combination thereof as defined herein to a patient in need thereof.

The present invention will now be illustrated with reference to the following figures and examples.

### Brief description of the figures

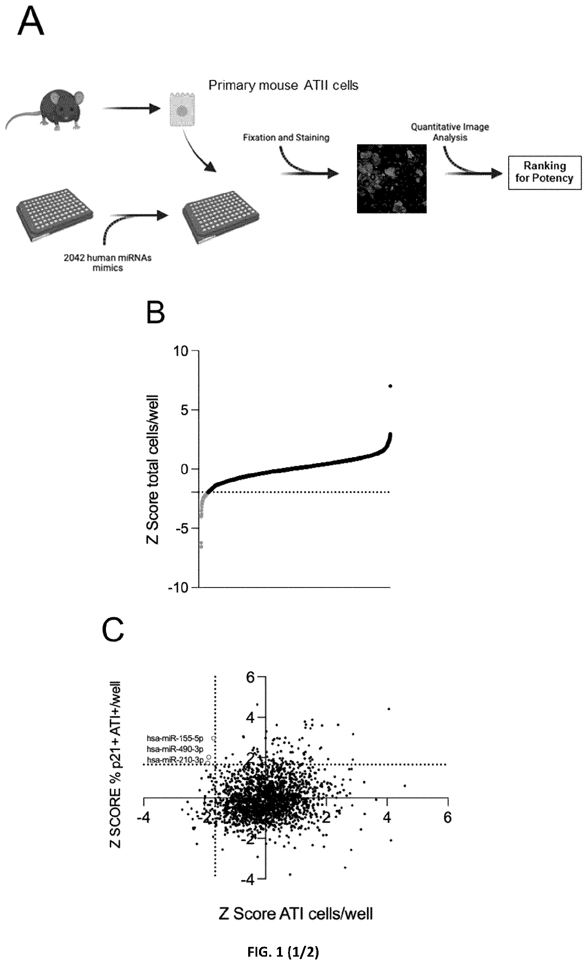

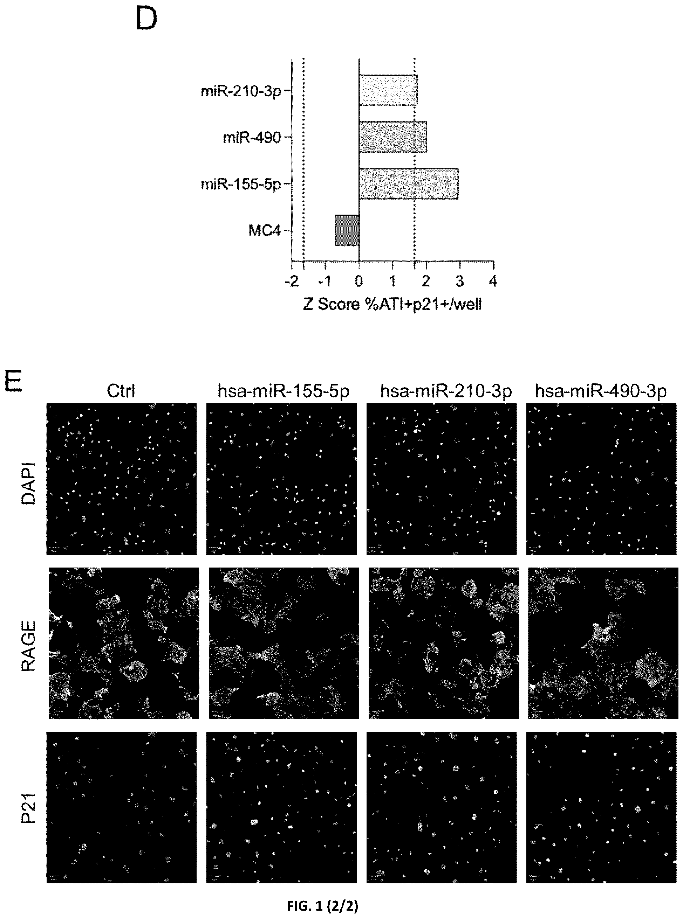

Figure 1. Human miRNA screening in primary mouse ATII cells for ATII senescence and ATII to ATItrans-differentiation. A. Screening workflow. Briefly 2042 human miRNA mimics were reverse-transfected (final concentration 25nM) into primary mouse ATII cells and at 72 hours after transfection cell were fixed in PFA and immune-stained for Rage (marker of ATI cells, to analyze the trans-differentiation) and P21 (marker of senescence). Plates were imaged and images were analyzed by counting the total cell number, the percentage of p21 and percentage of Rage positive cells. All non-toxic miRNAs were ranked for potency in either increasing cellular senescence (increasing the percentage of ATI/P21 positive cells) and blocking ATII to ATI trans-differentiation (decreasing of Rage positive cells percentage). B. Chart showing Z-Score of the total number of cells/well. MiRNAs corresponding to round gray dots are considered significantly toxic (Z≤-1.96, P≤0.05). C. Chart showing Z-Score of percentage of ATI/P21 positive cells. In particular, miRNAs corresponding to round white dots are considered significantly increasing the percentage of P21 positive cells/well (Z≥1.65, P≤0.10, dashed lines) and reducing the percentage of Rage positive cells, so inhibiting the trans-differentiation of ATII to ATI cells (Z≤-1.65, P≤0.10, dashed lines). Out of them, only 3 miRNAs (hsa-miR-155-5p, hsa-miR-210-3p and hsa-miR-490-3p) were simultaneously increasing cellular senescence (Zscore % ATI/P21 positive cells ≥1.65, p≤0.10). D. Image quantification, Z-Score of the percentage of ATI+P21+ cells/well for selected miRNAs and negative control (MC4, cel-miR-231-3p) are plotted. E. Representative immunofluorescence images of selected miRNAs that increasing cellular senescence, as it can be appreciated from the images of the single channel for P21 marker in bottom part of the panel, while decreasing ATII to ATI transdifferentiation, here represented by the total number of Rage (AT1) positive cells.

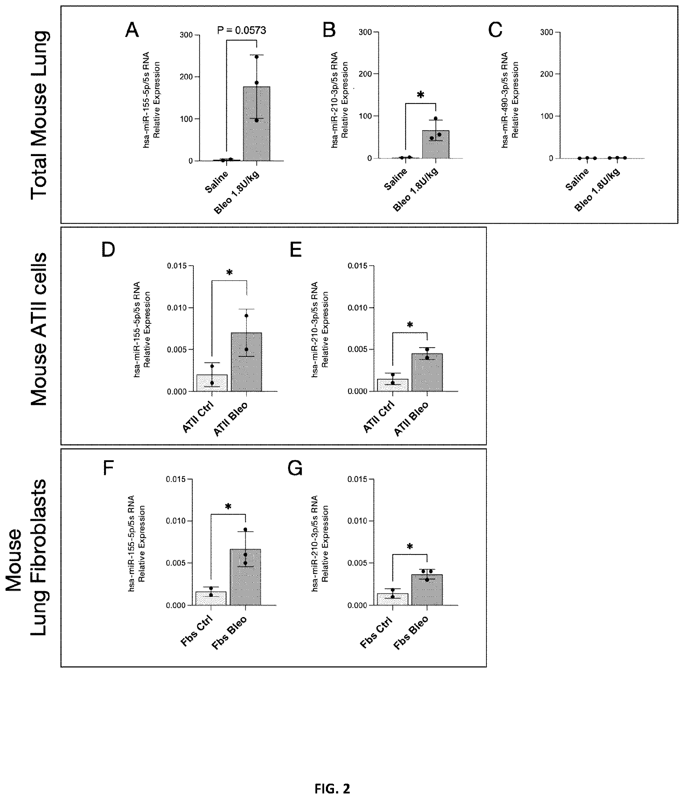

Figure 2. Two out of three selected miRNAs present high endogenous expression levels in bleomycin treated mice. A, B and C. Expression of selected miRNAs in total lung harvested from mice treated with saline (used as Ctrl) and mice treated with bleomycin. Specifically, mice were anesthetized by isoflurane and then, 60µl of bleomycin (1.8U/kg) were intratracheally injected using intubation method in mice. After 21 days from the injection, in the maximum development of lung fibrosis, mice were sacrificed. Graphs show an increase of endogenous expression of miR-155-5p (A) and miR-210-3p (B) in lung upon bleomycin treatment compared to the saline one. Whereas miR-490-3p isn't expressed in lung neither in sham nor damaged condition (C). D and E. Relative expression of selected miRNA (miR-155-5p and miR-210-3p) in ATII cells isolated from untreated and bleomycin treated mice. Mice were intratracheally injected with 1.8U/kg of bleomycin and the ATII cells were isolated upon 21 days from the injection. Graphs show an increase of endogenous levels of miR-155-5p (D) and miR-210-3p (E) in ATII isolated from bleomycin treated mice compared to untreated mice. F and G. Relative expression of selected miRNAs in murine alveolar fibroblasts isolated after 21 days from intratracheally injection of bleomycin and from untreated mice. As it can be evident from the graphs in figure 2F and 2G, both the expression of miR-155-5p (D) and miR-210-3p increase upon bleomycin treatment in alveolar fibroblasts. All endogenous expression levels of selected miRNAs were normalized to the 5S ribosomal RNA expression levels. Data were analyzed by one tailed unpaired t-test and statistical significance is reported as follows: *P≤0.05.

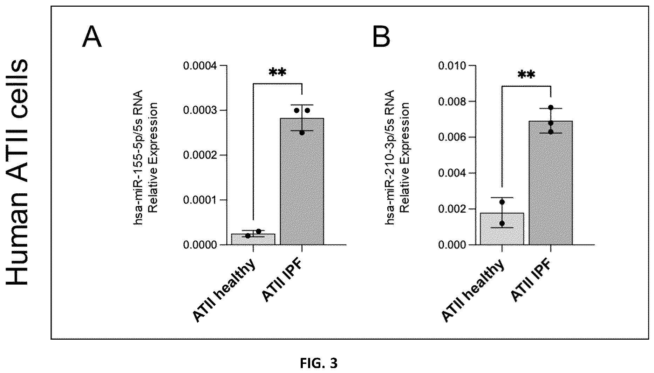

Figure 3. The selected miRNAs are more expressed in pathological conditions. A and B. Relative expression of miR-155-5p and miR-210-3p respectively in human ATII cells isolated from healthy patients and IPF patients. ATII cells were isolated from pieces of lung harvested from non-fibrotic and IPF patients. Charts show that the expression of selected miRNAs is significantly higher in ATII cells isolated from IPF patients compared to heathy one. All endogenous expression levels of selected miRNAs were normalized to the levels of 5S ribosomal RNA used as endogenous standard housekeeping. Data from 3 independent experiments/conditions were analyzed by One-Way ANOVA followed by Dunnet correction for multiple comparisons and statistical significance is reported as follows: ** P≤0,005.

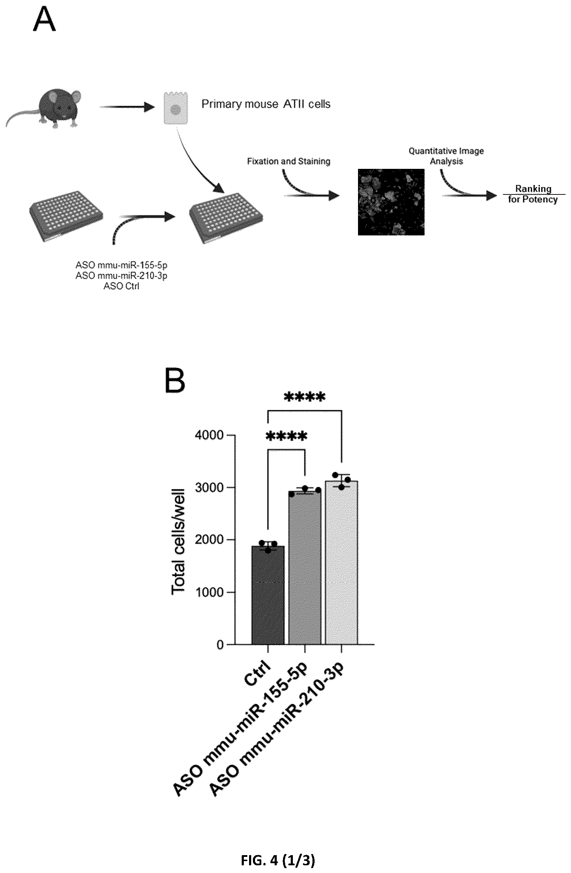

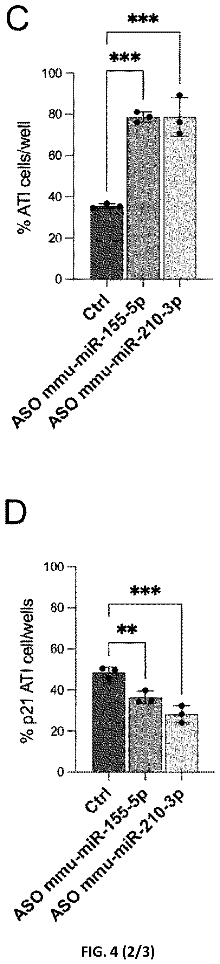

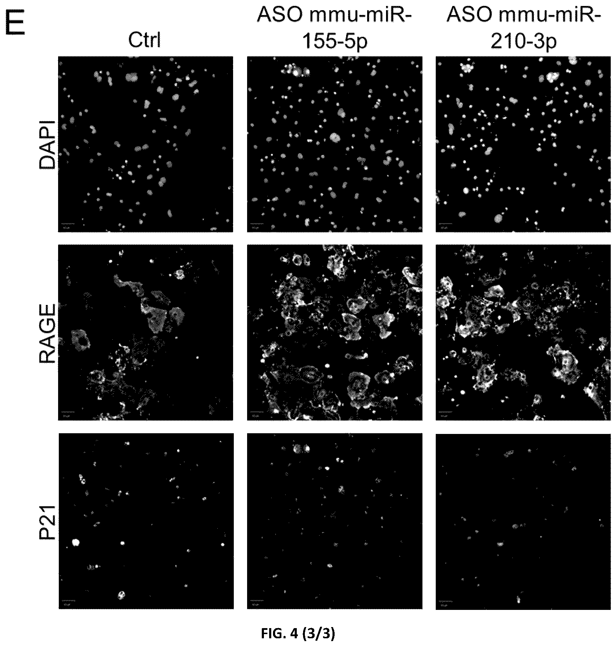

Figure 4. Effect of ASO-hsa-miRNAs on ATII cells isolated from bleomycin treated mice. A. Experimental workflow. ASO (antisense oligonucleotide) against selected two miRNAs (mmu-miR-155-5p and mmu-miR-210-3p) were reverse-transfected (final concentration 30nM) into ATII cells isolated from bleomycin treated mice (injection method, timing and dose are reported in the previous sections). After 72 hours from ASO transfection cells were fixed in PFA and stained for Rage (ATI cells), P21 (Senescent cells) and Hoechst (total cells). Plates were imaged and images were analyzed by counting the total cell number, the percentage of P21/Rage positive cells and the percentage of Rage positive cells. B. Quantification of total cell number of ATII cells/well. C. Quantification of the percentage of Rage positive cells over total number of cells per well. D. Quantification of the percentage of P21/Rage double positive cells over total number of cells per well. Data were analyzed by One-Way ANOVA followed by Dunnet correction for multiple comparisons and statistical significance is reported as it follows: ****P≤0.0001, ***P≤0.0005, ** P≤0.0075. E. Representative immunofluorescence images of bleomycin ATII cells treated with ASO against selected miRNAs. Immunofluorescence images show the effect of ASO in promoting ATII to ATI trans-differentiation as illustrated in the images for the single marker Rage and in reducing cellular senescence (Images for P21 senescence marker).

### EXAMPLES

### Example 1

### Primary Screening Results

Inventors performed a cell-based phenotypic high throughput screening (HTS) assay on 2042 human miRNA mimics for their efficacy in increasing cellular senescence and inhibiting ATII to ATI trans-differentiation in primary alveolar mouse epithelial cells. They identified three miRNA mimics that significantly (ZScore≥1.65, P≤0.10) increased cellular senescence and decreased (ZScore≤1.65, P≤0.10) ATII to ATI trans-differentiation. Inventors isolated and cultured mouse alveolar ATII cells, obtaining a purity ≥90%. Isolated cells were reverse transfected with an arrayed library of 2042 human miRNA mimics at 25nM final concentration (Horizon Discovery) (Experimental scheme in Figure 1A). In particular, 5µl of miRNA mimic (250nM) were spotted by a Hamilton Starlet Liquid Handler on the bottom of 384-wells-phenoplate (PerkinElmer #6057300) previously coated in fibronectin/gelatin. Immediately after a mixture composed of 15µl of Optimem (Gibco) and 0.15 lipofectamine RNAimax was added to each well, mixed and incubated for 30 minutes at room temperature. After 30 minutes of incubation, 15.000 cells diluted in a final volume of 30µl were added to each well. 72 hours post transfection the cells were fixed and stained for Rage (to label ATI cells and track the trans-differentiation), P21 (marker of senescence) and Hoechst 3342 (nuclei). An average of 1000 cells/condition have been imaged by automated fluorescence microscopy (Operetta CLS microscope) and a complete phenotypic profiling has been performed for all treatments.

Inventors excluded toxic miRNAs, that significantly reduced the number of cells/well (Z-Score viability ≤ -1.96, P≤0.05, round grey dot in Figure 1B). Among the non-toxic miRNAs, Inventors selected three miRNAs simultaneously increasing the percentage of P21 positive ATI (RAGE positive) cells (Z≥1,65, P≤0,10) and reducing the percentage of ATI cells, thus inhibiting the trans-differentiation (Z≤-1,65, P≤0,10). According to these criteria, three miRNAs were identified: hsa-miR-155-5p, hsa-miR-210-3p and hsa-miR490-3p respectively (Figure 1C and **Table 1**). Representative images and data of ATII cells transfected with the selected miRNAs are reported in Figure 1D and 1E, where it is possible to appreciate a clear increase in cellular senescence (p21 positivity) associated with a decrease in the total number of ATI cells/well.

**Table 1. Human miRNA sequences**

| ***miRNA*** | ***miRbase ID*** | ***Mature sequence 5'-3'*** | ***miRbase ID*** | ***Pre-miRNA sequence 5'-3'*** |
|---|---|---|---|---|
| hsa-miR-155-5p | MIMAT000 0646 | UUAAUGCUAAUCGUGA UAGGGGU (SEQ ID NO. 1) | MI00006 81 | CUGUUAAUGCUAA UCGUGAUAGGGGU UUUUGCCUCCAACU GACUCCUACAUAUU AGCAUUAACAG (SEQ ID NO. 2) |
| hsa-miR-210-3p | MIMAT000 0267 | CUGUGCGUGUGACAGC GGCUGA (SEQ ID NO. 3) | MI00002 86 | ACCCGGCAGUGCCU CCAGGCGCAGGGCA GCCCCUGCCCACCG CACACUGCGCUGCC CCAGACCCACUGUG CGUGUGACAGCGGC UGAUCUGUGCCUGG GCAGCGCGACCC (SEQ ID NO. 4) |
| hsa-miR-490-3p | MIMAT000 2806 | CAACCUGGAGGACUCC AUGCUG (SEQ ID NO. 5) | MI00031 25 | UGGAGGCCUUGCUG GUUUGGAAAGUUC AUUGUUCGACACCA UGGAUCUCCAGGUG GGUCAAGUUUAGA GAUGCACCAACCUG GAGGACUCCAUGCU GUUGAGCUGUUCA (SEQ ID NO. 6) |

### Endogenous expression levels of selected miRNAs in the total lung of bleomycin-treated mice

The overall aim of this research is to use miRNAs as potential target for the development of anti-fibrotic anti-miRNA-based therapies. Therefore, the assessment of endogenous miRNA levels in pathological conditions is essential to evaluate the potential role of selected miRNAs in the development and progression of IPF, and consequently therapeutical impact of selected miRNA inhibition. Accordingly, the inventors quantified the relative expression of selected miRNAs in total lung harvested from saline and bleomycin treated mice. According to the American Thoracic Society, the mouse model of bleomycin induced lung fibrosis represents the most reliable animal model for the preclinical assessment of potential therapies for pulmonary fibrosis ¹³. Briefly, C57BL/6 mice were anesthetized using isoflurane and then, intubated and intra-tracheal injected either, with 60 µl of saline (Ctrl group) or/with 60 µl of bleomycin (1.8U/kg final concentration). After 21 days from the injection, mice were sacrificed, and lungs were harvested and snap frozen in liquid nitrogen. RNA extraction from frozen lung tissues was performed using the miRNeasy Mini Kit (Qiagen). Each tissue sample was homogenized and lysed in 1ml of Trizol with magnalyzer. RNA was retrotranscribed using miRCURY LNA RT kit (Qiagen) and the endogenous expression of selected miRNAs was quantified by miRCURY LNA miRNA PCR Assay (Qiagen), using probes designed for murine sequences of selected miRNAs; reported in Table 2. Data were expressed as miRNA levels relative to the levels of 5S ribosomal RNA used as endogenous standard housekeeping.

Inventors observed that the endogenous expression of selected miRNAs resulted to be higher in bleomycin treated lungs compared to saline one (Figure 2 A and B). Regarding miR-490-3p; there was no expression in murine total lung neither in saline nor in bleomycin condition (Figure 2C) and for this reason it was not followed up further.

**Table 2. Murine miRNA sequences**

| ***miRNA*** | ***miRbase ID*** | ***Mature sequence 5'-3'*** | ***miRbase ID*** | ***Pre-miRNA sequence 5'-3'*** |
|---|---|---|---|---|
| mmu-miR-155-5p | MIMAT000 0165 | UUAAUGCUAAUUGUGA UAGGGGU (SEQ ID NO. 7) | MI00001 77 | CUGUUAAUGCUAA UUGUGAUAGGGGU UUUGGCCUCUGACU GACUCCUACCUGUU AGCAUUAACAG (SEQ ID NO. 8) |
| mmu-miR-210-3p | MIMAT000 0658 | CUGUGCGUGUGACAGC GGCUGA (SEQ ID NO. 3) | MI00006 95 | CCGGGGCAGUCCCU CCAGGCUCAGGACA GCCACUGCCCACCG |
| | | | | CACACUGCGUUGCU CCGGACCCACUGUG CGUGUGACAGCGGC UGAUCUGUCCCUGG GCAGCGCGAACC (SEQ ID NO. 9) |
| mmu-miR-490-3p | MIMAT000 3780 | CAACCUGGAGGACUCC AUGCUG (SEQ ID NO. 5) | MI00050 02 | UGGAAAGUUCAUU GUUCGACACCAUGG AUCUCCAGGUGGGU CACGUUUAGAUAU ACACCAACCUGGAG GACUCCAUGCUGUU GA (SEQ ID NO. 10) |

### Example 2

### Endogenous expression levels of selected miRNAs in the ATII cells isolated from the lung of bleomycin-treated mice

Inventors decided to investigate the different expression of selected miRNAs upon bleomycin treatment in the main cell types known to play an active role in the development of IPF. We treated adult C57BL/6 mice with Bleomycin, as described above. After 21 days from the injection, we isolated ATII cells, Ctrl ATII cells were isolated from saline untreated mice. To quantify the expression of selected miRNAs, RNA extraction was performed using miRNeasy Micro Kit (Qiagen). Then, samples were retrotranscribed using miRCURY LNA RT kit (Qiagen) and the endogenous expression of selected miRNAs was quantified by miRCURY LNA miRNA PCR Assay (Qiagen), using probes designed on the base on murine miRNAs sequences reported in **Table 2**. Data were expressed as miRNA levels relative to the levels of 5S ribosomal RNA used as endogenous standard housekeeping.

As shown in figure 2 D and E, there is an increase of the endogenous expression levels of mmumiR-155-5p and mmu-miR-210-3p in ATII cells isolated from bleomycin treated mice compared to untreated one.

### Example 3

### Endogenous expression levels of selected miRNAs in alveolar fibroblasts isolated from the lung of bleomycin-treated mice

Inventors quantified the endogenous expression levels of selected miRNAs also in alveolar fibroblasts isolated from the distal portion of the lungs belonging to saline (Ctrl) and bleomycin treated C57BL/6 mice. The isolation allows to obtain a purity ≥ 99 % after 2 days in culture in DMEM High Glucose 10% of FBS.

RNA was extracted from isolated fibroblasts using miRNeasy Micro Kit (Qiagen). Then, samples were retrotranscribed using miRCURY LNA RT kit (Qiagen) and the endogenous expression of selected miRNAs was quantified by miRCURY LNA miRNA PCR Assay (Qiagen), designed on murine miRNAs sequences reported in **Table 2.** Data were expressed as miRNA levels relative to the levels of 5S ribosomal RNA used as endogenous standard housekeeping. Also in alveolar fibroblasts, the expression of mmu-miR-155-5p and mmu-miR-210-3p resulted to be significantly upregulated upon bleomycin treatment compared to control condition. (Figure 2F and 2G).

### Example 4

### Endogenous expression levels of selected miRNAs in the ATII cells isolated from human IPF lungs

To elucidate the pathogenic role of selected miRNAs in the IPF lung disease. Inventors decided to quantify the expression of hsa-miR-155-5p and hsa-miR-210-3p in human ATII cells isolated from healthy patients and IPF patients. RNA isolation and cDNA generation was performed as described in the previous sections. The endogenous expression of selected miRNAs was quantified by miRCURY LNA miRNA PCR Assay (Qiagen), using probes designed on the base of human sequences for selected miRNAs reported in **Table 1.**

As shown in figure 3A and 3B, the expression of both miRNAs hsa-miR-155-5p and hsa-miR-210-3p increase in IPF ATII cells compared to ATII isolate from healthy controls. These results underline the important involvement of selected miRNAs in human IPF. MiRNA expression levels were expressed relative to the levels of 5S ribosomal RNA used as endogenous standard housekeeping.

### Example 5

### Effect of ASO (antisense oligonucleotide) on primary bleomycin alveolar epithelial cells

Given the involvement of the miR-155-5p and miR-210-3p in lung fibrotic remodelling and aging (Figure 2), Inventors proposed to use miRNAs as targets for the development of innovative anti-aging and pro-regenerative medication for IPF. The inventors decided to use LNA-modified ASO (LNA-ASOs) to target selected miRNAs in primary mouse ATII cells isolated from the lungs of bleomycin treated mice. ATII cells were isolated from C57BL/6 mice treated with 1.8U/kg of bleomycin at 21 days after bleomycin treatment. At this time point, ATII cells present higher levels of expression for selected miRNAs. In addition, diseased mouse ATII cells once isolated and cultured, are known to retain a limited ATII to ATI transdifferentiation capacity ⁶⁵. Similar evidence was shown on human ATII cells isolated from IPF lungs ⁶⁶. The aim of this experiment was to prove that LNAs, targeting selected miRNAs, were able to downregulate them and both reduce cellular senescence and restore effective ATII to ATI trans-differentiation in diseased ATII cells.

ASOs against selected miRNAs were designed and produced by QIAGEN, based on the sequence of miRNAs reported in Table 2. Sequences of said ASOs are 5'-GCTGTCACACGCACA-3' (SEQ ID NO.11) and 5'-TCACAATTAGCATTA-3' (SEQ ID NO.12). ASOs present some modifications in order to stabilize the structure, like the use of LNA nucleotides and a dimension between 13-16 nucleotides to be useful also in vivo. In particular, 7,5µl of ASO (500nM) were spotted on the bottom of 96-wells-phenoplate (PerkinElmer #6057300). Immediately after a mixture composed of 42µl of Optimem (Gibco) and 0.5 µl lipofectamine RNAimax was added to each well, mixed and incubated for 30 minutes at room temperature. After effective incubation, 50.000 cells diluted in a final volume of 100µl were added to each well. The assay was stopped at 72 hours post transfection (experimental scheme in Figure 4A). Finally, cells were stained with fluorescent probe for Rage (to label ATI cells and see the ATII to ATI trans-differentiation), P21 (marker of senescence) and Hoechst 3342 (nuclei). An average of 1000 cells/condition have been imaged by automated fluorescence microscopy (Operetta CLS microscope) and a complete phenotypic profiling has been performed for all treatments.

We found that both ASOs effectively restored ATII to ATI trans-differentiation while inhibiting cellular senescence in ATII cell isolated from bleomycin treated lungs (Figure 4B and 4C). In particular, ASOs increase the number of total cells, the percentage of Rage positive cells and decrease the percentage of P21 positive cells compared to the bleomycin ATII cells transfected with ASO Negative Ctrl (Figure 4D).

These results together strongly support the role of selected miRNAs in the impairment of ATII's capability to trans-differentiate into ATI cells and in promoting ATII cellular senescence. Whereas the use of ASOs targeting the selected miRNAs effectively restored ATII to ATI trans-differentiation capacity and reduced cellular senescence in diseased ATII cells.

### Conclusions

These data strongly support the therapeutic value of ASOs targeting the selected miRNAs in treating respiratory disease characterized by senescence of alveolar epithelial type II and/or type I cells and/or loss of alveolar epithelial type II to type I trans-differentiation capacity and/or with loss of lung regenerative capacity, leading to lung fibrotic remodelling.

### BIBLIOGRAPHY

1 Ley, B. & Collard, H. R. Epidemiology of idiopathic pulmonary fibrosis. Clin Epidemiol 5, 483-492, doi:10.2147/CLEP.S54815 (2013).
2 Barkauskas, C. E. et al. Type 2 alveolar cells are stem cells in adult lung. J Clin Invest 123, 3025-3036, doi:10.1172/JCI68782 (2013).
3 Desai, T. J., Brownfield, D. G. & Krasnow, M. A. Alveolar progenitor and stem cells in lung development, renewal and cancer. Nature 507, 190-194, doi:10.1038/nature12930 (2014).
4 Rock, J. R. & Hogan, B. L. Epithelial progenitor cells in lung development, maintenance, repair, and disease. Annu Rev Cell Dev Biol 27, 493-512, doi:10.1146/annurev-cellbio-100109-104040 (2011).
5 Adams, T. S. et al. Single-cell RNA-seq reveals ectopic and aberrant lung-resident cell populations in idiopathic pulmonary fibrosis. Sci Adv 6, eaba1983, doi:10.1126/sciadv.aba1983 (2020).
6 Basil, M. C. et al. Human distal airways contain a multipotent secretory cell that can regenerate alveoli. Nature 604, 120-126, doi:10.1038/s41586-022-04552-0 (2022).
7 Habermann, A. C. et al. Single-cell RNA sequencing reveals profibrotic roles of distinct epithelial and mesenchymal lineages in pulmonary fibrosis. Sci Adv 6, eaba1972, doi:10.1126/sciadv.aba1972 (2020).
8 Kobayashi, Y. et al. Persistence of a regeneration-associated, transitional alveolar epithelial cell state in pulmonary fibrosis. Nat Cell Biol 22, 934-946, doi:10.1038/s41556-020-0542-8 (2020).
9 Strunz, M. et al. Alveolar regeneration through a Krt8+ transitional stem cell state that persists in human lung fibrosis. Nat Commun 11, 3559, doi:10.1038/s41467-020-17358-3 (2020).
10 Jiang, P. et al. Ineffectual Type 2-to-Type 1 Alveolar Epithelial Cell Differentiation in Idiopathic Pulmonary Fibrosis: Persistence of the KRT8(hi) Transitional State. Am J Respir Crit Care Med 201, 1443-1447, doi:10.1164/rccm.201909-1726LE (2020).
11 Choi, J. et al. Inflammatory Signals Induce AT2 Cell-Derived Damage-Associated Transient Progenitors that Mediate Alveolar Regeneration. Cell Stem Cell 27, 366-382 e367, doi:10.1016/j.stem.2020.06.020 (2020).
12 Wang, F. et al. Regulation of epithelial transitional states in murine and human pulmonary fibrosis. J Clin Invest 133, doi:10.1172/JCI165612 (2023).
13 Jenkins, R. G. et al. An Official American Thoracic Society Workshop Report: Use of Animal Models for the Preclinical Assessment of Potential Therapies for Pulmonary Fibrosis. Am J Respir Cell Mol Biol 56, 667-679, doi:10.1165/rcmb.2017-0096ST (2017).
14 Schafer, M. J. et al. Cellular senescence mediates fibrotic pulmonary disease. Nat Commun 8, 14532, doi:10.1038/ncomms14532 (2017).
15 Aoshiba, K. et al. Senescence-associated secretory phenotype in a mouse model of bleomycin-induced lung injury. Exp Toxicol Pathol 65, 1053-1062, doi:10.1016/j.etp.2013.04.001 (2013).
16 Chen, X. et al. Epithelial cell senescence induces pulmonary fibrosis through Nanogmediated fibroblast activation. Aging (Albany NY) 12, 242-259, doi:10.18632/aging.102613 (2019).
17 Martinez, F. J. et al. Idiopathic pulmonary fibrosis. Nat Rev Dis Primers 3, 17074, doi:10.1038/nrdp.2017.74 (2017).
18 Redente, E. F. et al. Tumor necrosis factor-alpha accelerates the resolution of established pulmonary fibrosis in mice by targeting profibrotic lung macrophages. Am J Respir Cell Mol Biol 50, 825-837, doi:10.1165/rcmb.2013-03860C (2014).
19 Hecker, L. et al. Reversal of persistent fibrosis in aging by targeting Nox4-Nrf2 redox imbalance. Sci Transl Med 6, 231ra247, doi:10.1126/scitranslmed.3008182 (2014).
20 Nambiar, A. et al. Senolytics dasatinib and quercetin in idiopathic pulmonary fibrosis: results of a phase I, single-blind, single-center, randomized, placebo-controlled pilot trial on feasibility and tolerability. EBioMedicine 90, 104481, doi:10.1016/j.ebiom.2023.104481 (2023).
21 Justice, J. N. et al. Senolytics in idiopathic pulmonary fibrosis: Results from a first-inhuman, open-label, pilot study. EBioMedicine 40, 554-563, doi:10.1016/j.ebiom.2018.12.052 (2019).
22 Eulalio, A. et al. Functional screening identifies miRNAs inducing cardiac regeneration. Nature 492, 376-381, doi:10.1038/nature11739 (2012).
23 Filipowicz, W., Bhattacharyya, S. N. & Sonenberg, N. Mechanisms of post-transcriptional regulation by microRNAs: are the answers in sight? Nat Rev Genet 9, 102-114, doi:10.1038/nrg2290 (2008).
24 Ghildiyal, M. & Zamore, P. D. Small silencing RNAs: an expanding universe. Nat Rev Genet 10, 94-108, doi:10.1038/nrg2504 (2009).
25 Boateng, E. & Krauss-Etschmann, S. miRNAs in Lung Development and Diseases. Int J Mol Sci 21, doi:10.3390/ijms21082765 (2020).
26 Harris, K. S., Zhang, Z., McManus, M. T., Harfe, B. D. & Sun, X. Dicer function is essential for lung epithelium morphogenesis. Proc Natl Acad Sci U S A 103, 2208-2213, doi:10.1073/pnas.0510839103 (2006).
27 Stolzenburg, L. R. & Harris, A. The role of microRNAs in chronic respiratory disease: recent insights. Biol Chem 399, 219-234, doi:10.1515/hsz-2017-0249 (2018).
28 Montgomery, R. L. et al. MicroRNA mimicry blocks pulmonary fibrosis. EMBO Mol Med 6, 1347-1356, doi:10.15252/emmm.201303604 (2014).
29 Marts, L. T., Green, D. E., Mills, S. T., Murphy, T. & Sueblinvong, V. MiR-21-Mediated Suppression of Smad7 Induces TGFbeta1 and Can Be Inhibited by Activation of Nrf2 in Alcohol-Treated Lung Fibroblasts. Alcohol Clin Exp Res 41, 1875-1885, doi:10.1111/acer.13496 (2017).
30 Cushing, L., Kuang, P. & Lu, J. The role of miR-29 in pulmonary fibrosis. Biochem Cell Biol 93, 109-118, doi:10.1139/bcb-2014-0095 (2015).
31 Liang, C. et al. The anti-fibrotic effects of microRNA-153 by targeting TGFBR-2 in pulmonary fibrosis. Exp Mol Pathol 99, 279-285, doi:10.1016/j.yexmp.2015.07.011 (2015).
32 Baumann, V. & Winkler, J. miRNA-based therapies: strategies and delivery platforms for oligonucleotide and non-oligonucleotide agents. Future Med Chem 6, 1967-1984, doi:10.4155/fmc.14.116 (2014).
33 Wang, X. et al. Adenovirus-mediated shRNAs for co-repression of miR-221 and miR-222 expression and function in glioblastoma cells. Oncol Rep 25, 97-105 (2011).
34 Ebert, M. S., Neilson, J. R. & Sharp, P. A. MicroRNA sponges: competitive inhibitors of small RNAs in mammalian cells. Nat Methods 4, 721-726, doi:10.1038/nmeth1079 (2007).
35 Lavenniah, A. et al. Engineered Circular RNA Sponges Act as miRNA Inhibitors to Attenuate Pressure Overload-Induced Cardiac Hypertrophy. Mol Ther 28, 1506-1517, doi:10.1016/j.ymthe.2020.04.006 (2020).
36 Haraguchi, T., Ozaki, Y. & Iba, H. Vectors expressing efficient RNA decoys achieve the long-term suppression of specific microRNA activity in mammalian cells. Nucleic Acids Res 37, e43, doi:10.1093/nar/gkp040 (2009).
37 Hooykaas, M. J. G. et al. RNA accessibility impacts potency of Tough Decoy microRNA inhibitors. RNA Biol 15, 1410-1419, doi:10.1080/15476286.2018.1537746 (2018).
38 Meng, L. et al. Small RNA zippers lock miRNA molecules and block miRNA function in mammalian cells. Nat Commun 8, 13964, doi:10.1038/ncomms13964 (2017).
39 Lima, J. F., Cerqueira, L., Figueiredo, C., Oliveira, C. & Azevedo, N. F. Anti-miRNA oligonucleotides: A comprehensive guide for design. RNA Biol 15, 338-352, doi:10.1080/15476286.2018.1445959 (2018).
40 Szczepanek, J., Skorupa, M. & Tretyn, A. MicroRNA as a Potential Therapeutic Molecule in Cancer. Cells 11, doi:10.3390/cells11061008 (2022).
41 Dasgupta, I. & Chatterjee, A. Recent Advances in miRNA Delivery Systems. Methods Protoc 4, doi:10.3390/mps4010010 (2021).
42 Zununi Vahed, S., Salehi, R., Davaran, S. & Sharifi, S. Liposome-based drug codelivery systems in cancer cells. Mater Sci Eng C Mater Biol Appl 71, 1327-1341, doi:10.1016/j.msec.2016.11.073 (2017).
43 O'Neill, C. P. & Dwyer, R. M. Nanoparticle-Based Delivery of Tumor Suppressor microRNA for Cancer Therapy. Cells 9, doi:10.3390/cells9020521 (2020).
44 Herrera-Carrillo, E., Liu, Y. P. & Berkhout, B. Improving miRNA Delivery by Optimizing miRNA Expression Cassettes in Diverse Virus Vectors. Hum Gene Ther Methods 28, 177-190, doi:10.1089/hgtb.2017.036 (2017).
45 Huang, X., Le, Q. T. & Giaccia, A. J. MiR-210--micromanager of the hypoxia pathway. Trends Mol Med 16, 230-237, doi:10.1016/j.molmed.2010.03.004 (2010).
46 Bruning, U. et al. MicroRNA-155 promotes resolution of hypoxia-inducible factor 1alpha activity during prolonged hypoxia. Mol Cell Biol 31, 4087-4096, doi:10.1128/MCB.01276-10 (2011).
47 Li, H., Zhao, X., Shan, H. & Liang, H. MicroRNAs in idiopathic pulmonary fibrosis: involvement in pathogenesis and potential use in diagnosis and therapeutics. Acta Pharm Sin B 6, 531-539, doi:10.1016/j.apsb.2016.06.010 (2016).
48 Chen, Y. et al. Inhibition of miR-155-5p Exerts Anti-Fibrotic Effects in Silicotic Mice by Regulating Meprin alpha. Mol Ther Nucleic Acids 19, 350-360, doi:10.1016/j.omtn.2019.11.018 (2020).
49 Bodempudi, V. et al. miR-210 promotes IPF fibroblast proliferation in response to hypoxia. Am J Physiol Lung Cell Mol Physiol 307, L283-294, doi:10.1152/ajplung.00069.2014 (2014).
50 Dinami, R. et al. miR-155 drives telomere fragility in human breast cancer by targeting TRF1. Cancer Res 74, 4145-4156, doi:10.1158/0008-5472.CAN-13-2038 (2014).
51 Brummelkamp, T. R., Bernards, R. & Agami, R. A system for stable expression of short interfering RNAs in mammalian cells. Science 296, 550-553, doi:10.1126/science.1068999 (2002).
52 Lee, N. S. et al. Expression of small interfering RNAs targeted against HIV-1 rev transcripts in human cells. Nat Biotechnol 20, 500-505, doi:10.1038/nbt0502-500 (2002).
53 Miyagishi, M. & Taira, K. U6 promoter-driven siRNAs with four uridine 3' overhangs efficiently suppress targeted gene expression in mammalian cells. Nat Biotechnol 20, 497-500, doi:10.1038/nbt0502-497 (2002).
54 Paddison, P. J., Caudy, A. A., Bernstein, E., Hannon, G. J. & Conklin, D. S. Short hairpin RNAs (shRNAs) induce sequence-specific silencing in mammalian cells. Genes Dev 16, 948-958, doi:10.1101/gad.981002 (2002).
55 Paul, C. P., Good, P. D., Winer, I. & Engelke, D. R. Effective expression of small interfering RNA in human cells. Nat Biotechnol 20, 505-508, doi:10.1038/nbt0502-505 (2002).
56 Yu, J. Y., DeRuiter, S. L. & Turner, D. L. RNA interference by expression of shortinterfering RNAs and hairpin RNAs in mammalian cells. Proc Natl Acad Sci USA 99, 6047-6052, doi:10.1073/pnas.092143499 (2002).
57 Sui, G. et al. A DNA vector-based RNAi technology to suppress gene expression in mammalian cells. Proc Natl Acad Sci U S A 99, 5515-5520, doi:10.1073/pnas.082117599 (2002).
58 Kluiver, J. et al. Generation of miRNA sponge constructs. Methods 58, 113-117, doi:10.1016/j.ymeth.2012.07.019 (2012).
59 Misir, S., Wu, N. & Yang, B. B. Specific expression and functions of circular RNAs. Cell Death Differ 29, 481-491, doi:10.1038/s41418-022-00948-7 (2022).
60 Bak, R. O., Hollensen, A. K., Primo, M. N., Sorensen, C. D. & Mikkelsen, J. G. Potent microRNA suppression by RNA Pol II-transcribed 'Tough Decoy' inhibitors. RNA 19, 280-293, doi: 10.1261/rna.034850.112 (2013).
61 Xie, J. et al. Long-term, efficient inhibition of microRNA function in mice using rAAV vectors. Nat Methods 9, 403-409, doi:10.1038/nmeth.1903 (2012).
62 Jeong, D. et al. miR-25 Tough Decoy Enhances Cardiac Function in Heart Failure. Mol Ther 26, 718-729, doi:10.1016/j.ymthe.2017.11.014 (2018).
63 Rindler, T. N. et al. Efficient Transduction of Alveolar Type 2 Cells with Adeno-associated Virus for the Study of Lung Regeneration. Am J Respir Cell Mol Biol 65, 118-121, doi:10.1165/rcmb.2021-0049LE (2021).
64 van Lieshout, L. P. et al. A Novel Triple-Mutant AAV6 Capsid Induces Rapid and Potent Transgene Expression in the Muscle and Respiratory Tract of Mice. Mol Ther Methods Clin Dev 9, 323-329, doi:10.1016/j.omtm.2018.04.005 (2018).
65 Volpe, M. C. et al. Flt1 produced by lung endothelial cells impairs ATII cell transdifferentiation and repair in pulmonary fibrosis. Cell Death Dis 14, 437, doi:10.1038/s41419-023-05962-2 (2023).
66 Moimas, S. et al. miR-200 family members reduce senescence and restore idiopathic pulmonary fibrosis type II alveolar epithelial cell transdifferentiation. ERJ Open Res 5, doi:10.1183/23120541.00138-2019 (2019).

## Claims

1. An agent for use in promoting lung regeneration in a subject with a lung pathological condition, said agent being selected from the group consisting of:
- an inhibitor of miR-210-3p;
- an inhibitor of miR-155-5p;
or a combination thereof, preferably said miR-210-3p is human hsa-miR-210-3p and/or said miR-155-5p is human hsa-miR-155-5p.

2. An agent for use in the prevention and/or treatment of a lung pathological condition associated with senescence of alveolar epithelial type II and/or type I cells and/or with loss of alveolar epithelial type II to type I trans-differentiation capacity and/or with loss of lung regenerative capacity, said agent being selected from the group consisting of:
- an inhibitor of miR-210-3p;
- an inhibitor of miR-155-5p;
or a combination thereof, preferably said miR-210-3p is human hsa-miR-210-3p and/or said miR-155-5p is human hsa-miR-155-5p.

3. The agent for use according to claim 1 or 2, wherein the lung pathological condition is selected from the group consisting of: interstitial lung disease, chronic obstructive pulmonary disease, post-acute respiratory distress syndrome, and post-acute COVID-19 syndrome.

4. The agent for the use according to claim 3 wherein said lung pathological condition is an interstitial lung disease selected from the group consisting of idiopathic pulmonary fibrosis, non-specific interstitial pneumonia, desquamative interstitial pneumonia, respiratory bronchiolitis-ILD, cryptogenic organizing pneumonia and acute interstitial pneumonia.

5. The agent for use according to any one of previous claims wherein the lung pathological condition is idiopathic pulmonary fibrosis.

6. The agent for use according to any one of previous claims wherein said agent is used when the acute phase of the inflammatory response of said lung pathological condition is ceased.

7. The agent for use according to any one of previous claims, wherein said inhibitor is a nucleic acid molecule preferably selected from: a single-stranded or double-stranded nucleic acid molecule, an siRNA molecule, an shRNA molecule, an antisense oligonucleotide, a linear miRNA sponge, a circular RNA miRNA sponge, a miRNA-targeting Tough Decoy (TuD) RNA, and a miRZip, derivatives and mixtures thereof.

8. The agent for use according to claim 7, wherein said nucleic acid molecule, or a portion thereof, has sufficient complementarity to miR-210-3p and/or miR-155-5p, or a fragment thereof, to form a hybrid under physiological conditions and/or has at least 50%, 60%, 70%, 80%, 90%, 95%, 99% or 100% complementarity to a portion of a hsamiR-210-3p sequence and/or of a hsa-miR-155-5p sequence and/or comprises at least 7 nucleotides complementary to at least one sequence selected from SEQ ID NOs: 1-4, 7-9, or a variant or a fragment thereof, preferably complementary to the seed sequence of said miRNAs, said seed sequence being 5'-UGUGCGU-3' for miR-210-3p and 5'-UAAUGCU-3' for miR-155-5p, and/or binds to a miRNA comprising a nucleic acid that is at least 50%, 60%, 70%, 80%, 90%, 95%, 99% or 100% identical to any one of SEQ ID NOs: 1-4, 7-9.

9. The agent for use according to any one of previous claims, wherein:
the inhibitor of miR-210-3p comprises a nucleotide sequence comprising or consisting of the sequence 5'-GCTGTCACACGCACA-3' (SEQ ID NO 11) and/or the inhibitor comprises about 8 to about 30 nucleotides in length;
the inhibitor of miR-155-5p comprises a nucleotide sequence comprising or consisting of the sequence 5'- TCACAATTAGCATTA-3' (SEQ ID NO 12) and/or the inhibitor comprises about 8 to about 30 nucleotides in length;
and/or the inhibitor comprises at least 1 nucleotide, at least 2 nucleotides, at least 3 nucleotides, at least 4 nucleotides, at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, or at least 20 nucleotides at the 5' and/or at the 3' of said nucleotide sequence.

10. The agent for use according to any one of previous claims, wherein the inhibitor is an antisense oligonucleotide and it comprises at least one modified nucleotide, preferably the at least one modified nucleotide is a locked nucleic acid (LNA), an unlocked nucleic acid (UNA), an arabino nucleic acid (ABA), a bridged nucleic acid (BNA), and/or a peptide nucleic acid (PNA) and/or the inhibitor comprises at least one backbone modification, preferably a phosphorodiamidate morpholino oligomer (PMO) and/or phosphorothioate (PS) modification.

11. A nucleic acid coding for the at least one agent as defined in any one of claims 1 to 10 for use in the treatment and/or prevention of lung pathological conditions associated with senescence of alveolar epithelial type II and/or type I cells and/or with loss of alveolar epithelial type II to type I trans-differentiation capacity and/or with loss of lung regenerative capacity and/or for use in promoting lung regeneration in a subject with a lung pathological condition, wherein the lung pathological condition is preferably selected from the group consisting of interstitial lung disease, chronic obstructive pulmonary disease, post-acute respiratory distress syndrome, and post-acute COVID-19 syndrome, said interstitial lung disease being preferably selected from the group consisting of idiopathic pulmonary fibrosis, non-specific interstitial pneumonia, desquamative interstitial pneumonia, respiratory bronchiolitis-ILD, cryptogenic organizing pneumonia and acute interstitial pneumonia.

12. The agent for use according to any one of claims 1-10 or the nucleic acid for use according to claim 11, wherein said agent or nucleic acid is provided naked or within a delivery agent, preferably wherein the delivery agent comprises a vector, preferably a recombinant expression vector or a viral vector, a micelle, an exosome, a lipidoid, a lipoplex, an extracellular vesicle, a synthetic vesicle, a polymeric compound, a peptide, a protein, a cell, a nanoparticle mimic, a nanotube, a conjugate, nanoparticles, microparticles, a liposome or other biological or synthetic vesicle or material including lipid nanoparticles, polymer-based nanoparticles, polymer-lipid hybrid a nanoparticle, a microparticle, a microsphere, a liposome, a colloidal gold particle, a graphene composite, a cholesterol conjugate, a cyclodextrin complex, a polyethylenimine polymer, a lipopolysaccharide, a polypeptide, a polysaccharide, a lipopolysaccharide, a collagen, a pegylation of viral vehicle, or combinations thereof.

13. A vector, preferably a recombinant expression vector, comprising a coding sequence for the agent as defined in any of claims 1 to 10 or comprising the nucleic acid of claim 11, and/or expressing the agent as defined in any one of claims 1 to 10 or expressing the nucleic acid of claim 11, preferably under the control of a suitable promoter, for use in the treatment and/or prevention of lung pathological conditions associated with senescence of alveolar epithelial type II and/or type I cells and/or with loss of alveolar epithelial type II to type I trans-differentiation capacity and/or with loss of lung regenerative capacity and/or for use in promoting lung regeneration in a subject with a lung pathological condition, wherein the lung pathological condition is preferably selected from the group consisting of interstitial lung diseases, chronic obstructive pulmonary diseases, post-acute respiratory distress syndrome, and post-acute COVID-19 syndrome, said interstitial lung disease being preferably selected from the group consisting of idiopathic pulmonary fibrosis, non-specific interstitial pneumonia, desquamative interstitial pneumonia, respiratory bronchiolitis-ILD, cryptogenic organizing pneumonia and acute interstitial pneumonia,
preferably the vector is a viral or non-viral vector, preferably the viral vector is selected from adeno-associated virus (AAV) vectors of any capsid serotype, either natural or artificial, lentivirus vectors, adenoviral vector, retroviral vectors, alphaviral vectors, vaccinia virus vectors, herpes simplex virus (HSV) vectors, rabies virus vectors, and Sindbis virus vectors, preferably the adeno-associated virus (AAV) vector is AAV6, AAV6.2 or AAV6.2FF.

14. A recombinant adeno-associated virus (rAAV) particle comprising a nucleic acid encoding the at least one agent as defined in any one of claims 1-10, preferably the particle comprises a capsid derived from adeno-associated vectors AAV6, AAV6.2, AAV6.2FF, AAV8, AAV1, AAV2, AAV5, or AAV9, preferably wherein the nucleic acid is operably linked to a viral promoter or a tissue specific promoter for use in the treatment and/or prevention of lung pathological conditions associated with senescence of alveolar epithelial type II and/or type I cells and/or with loss of alveolar epithelial type II to type I trans-differentiation capacity and/or with loss of lung regenerative capacity and/or for use in promoting lung regeneration in a subject with a lung condition, wherein the pathological condition or the lung condition is preferably selected from the group of interstitial lung diseases, chronic obstructive pulmonary diseases, post-acute respiratory distress syndrome, and post-acute COVID-19 syndrome, said interstitial lung disease being preferably selected from the group consisting of idiopathic pulmonary fibrosis, non-specific interstitial pneumonia, desquamative interstitial pneumonia, respiratory bronchiolitis-ILD, cryptogenic organizing pneumonia and acute interstitial pneumonia.

15. A pharmaceutical composition for use in the treatment and/or prevention of lung pathological conditions associated with senescence of alveolar epithelial type II and/or type I cells and/or with loss of alveolar epithelial type II to type I trans-differentiation capacity and/or with loss of lung regenerative capacity and/or for use in promoting lung regeneration in a subject with a lung pathological condition, wherein the lung pathological condition is preferably selected from the group consisting of interstitial lung diseases, chronic obstructive pulmonary diseases, post-acute respiratory distress syndrome, and post-acute COVID-19 syndrome, said interstitial lung disease being preferably selected from the group consisting of idiopathic pulmonary fibrosis, non-specific interstitial pneumonia, desquamative interstitial pneumonia, respiratory bronchiolitis-ILD, cryptogenic organizing pneumonia and acute interstitial pneumonia, said pharmaceutical composition comprising an agent as defined in any one of claims 1-10 or 12, or the nucleic acid as defined in claim 11 or 12, or the vector as defined in claim 13, or a recombinant adeno-associated virus (rAAV) particle as defined in claim 14 and at least one pharmaceutically acceptable vehicle and/or excipient.
